(19)

**Europäisches Patentamt**
**European Patent Office**
**Office européen des brevets**

(11) **EP 4 130 296 A1**

(12) **EUROPEAN PATENT APPLICATION**

(43) Date of publication:
**08.02.2023 Bulletin 2023/06**

(21) Application number: **21189450.6**

(22) Date of filing: **03.08.2021**

(51) International Patent Classification (IPC):
***C12Q 1/6886*** *(2018.01)*

(52) Cooperative Patent Classification (CPC):
**C12Q 1/6886;** C12Q 2600/158; C12Q 2600/178

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Deutsches Krebsforschungszentrum - Stiftung des öffentlichen Rechts / Universität Heidelberg 69120 Heidelberg (DE)**

(72) Inventors:
• **Schrotz-King, Petra**
  **69120 Heidelberg (DE)**

• **Raut, Janhavi**
  **69120 Heidelberg (DE)**
• **Brenner, Hermann**
  **69120 Heidelberg (DE)**

(74) Representative: **Altmann Stößel Dick Patentanwälte PartG mbB Theodor-Heuss-Anlage 2 68165 Mannheim (DE)**

Remarks:
The complete document including Reference Table(s) and the Sequence Listing(s) can be downloaded from the EPO website

(54) **CANCER PREDICTION**

(57)    The present invention relates to a method for identifying a subject at risk of developing cancer, said method comprising (a) determining in a sample of said subject the amount of at least one miRNA selected from the list consisting of miR-19a-3p, let-7g-5p, miR-23a-3p, miR-92a-3p, miR-144-5p, and miR-27a-3p; (b) comparing said amount or amounts determined in step (a) to a reference or to references; and (c) identifying a subject at risk of developing cancer based on the result of comparing step (b), and to methods, kits, devices, and systems related thereto.

EP 4 130 296 A1

**Description**

The present invention relates to a method for identifying a subject at risk of developing cancer, said method comprising (a) determining in a sample of said subject the amount of at least one miRNA selected from the list consisting of miR-19a-3p, let-7g-5p, miR-23a-3p, miR-92a-3p, miR-144-5p, and miR-27a-3p; (b) comparing said amount or amounts determined in step (a) to a reference or to references; and (c) identifying a subject at risk of developing cancer based on the result of comparing step (b), and to methods, kits, devices, and systems related thereto.

Colorectal cancer (CRC) is the third most common incident cancer and the second leading cause of cancer mortality worldwide, accounting for 1.85 million incident cases and approximately 880,000 deaths in 2018. The disease burden can be decreased with population-based screening, which has been demonstrated to be effective in reducing mortality and potentially preventing the occurrence of CRC. Currently, colonoscopy is regarded as the gold standard method for early diagnosis of CRC, but its widespread use is limited by its invasive nature, dietary restriction requirement, and costs. While fecal immunochemical test for hemoglobin has been proven to be an effective, currently available non-invasive test to screen patients who are at average risk for the development of CRC, it has limited sensitivity to detect advanced adenomas or stage I CRCs. In order to maximize screening benefits and minimize harms and costs, alternative minimally invasive or non-invasive tests that can more accurately define low- and high-risk populations are needed. Risk models based on genetic susceptibility loci, alone or in combination with environmental risk factors have been increasingly propagated for risk stratification in CRC screening. However, the models used so far have generally yielded limited ability to distinguish between individuals with and without CRC and its precursors Kurlapska et al. (2015), Clin Genet, 88(3):234; Jeon et al. (2018) Gastroenterology 154(8):2152).

miRNAs are small, non-coding RNAs (~18-25 nucleotides in length) that regulate gene expression on a post-transcriptional level by degrading mRNA molecules or blocking their translation (Bartel (2004), Cell 2004;116:281). Hence, they play an essential role in the regulation of a large number of biological processes, including cancer. Under the standard nomenclature system, names are assigned to experimentally confirmed miRNAs. The prefix "mir" is followed by a dash and a number. The uncapitalized "mir-" refers to the pre-miRNA, while a capitalized "miR-" refers to the mature form. miRNAs with nearly identical sequences bar one or two nucleotides are annotated with an additional lower case letter. Species of origin is designated with a three-letter prefix, e.g. hsa for Homo sapiens (human). Two mature miRNAs originating from opposite arms of the same pre-miRNA are denoted with a -3p or - 5p suffix.

Circulating miRNAs are defined as miRNAs present in the cell-free component of body fluids like plasma, serum, and the like. Circulating miRNAs have been reported as aberrantly expressed in blood plasma or serum in different types of cancer, e.g. prostate, colorectal or esophageal carcinoma. Their most important advantages include the possibility to be measured repeatedly in a minimally invasive manner as well as their remarkable stability in plasma/serum.

In recent years, blood levels of microRNAs (miRNAs) have been linked to CRC development (Strubberg et al. (2017), Dis Model Mech, 10(3): 197; Schetter et al. (2012), Cancer Journal 18(3):244) and have consistently shown some potential at distinguishing CRC patients and controls free of colorectal neoplasms (Carter et al. (2017), Br J Cancer 116(6):762; Marcuello et al. (2019) Cancers 11(10): 1542; Rodriguez-Montes et al. (2014), Cir Esp, 92(10):654; Toiyama et al (2018) Biochim Biophys Acta Rev Cancer 1870(2):274; Sun et al. (2016), Oncotarget 7(10): 11434; Zanutto et al. (2020), Int J Cancer 146(4):1164; Wikberg et al. (2018) Cancer Med 7(5): 1697; Bader et al. (2020), IUBMB Life 72(2):275; Chang et al. (2016), Oncotarget 7(9):10663; Giraldez et al. (2013), Clin Gastroenterol Hepatol 11(6):681; Huang et al (2010), Int J Cancer 127(1): 118; Ng et al. (2009), Gut 58(10): 1375; Pan et al. (2017), Oncotarget 8(40):68317; Tan et al. (2019), Cancer Epidemiol 60:67; Toiyama et al. (2013), J Natl Cancer Inst 105(12):849; Vychytilova-Faltejskova et al. (2016), Carcinogenesis 37(10):941; Wang et al. (2014) PLoS One 9(4):e87451; Zanutto et al. (2014), Br J Cancer 110(4):1001; Zheng et al. (2014), Br J Cancer 111(10):198; Zhu et al. (2017), Oncotarget 8(10):17081;). It is, however, still unclear, if and to what extent, blood-based miRNA signatures might enable early CRC risk prediction.

Early prediction of cancer is deemed valuable since it is known that cancer risk can be mitigated by preventive measures, e.g. lifestyle changes (Erben et al. (2021), Cancer Prev Res 14(6): 649; Carr et al. (2020), Gastroenterology 159(1): 129 ; Carr et al. (2018), Gastroenterology 155(6): 1805).

In view of the above, there is still a need in the art for improved means and methods for predicting cancer. This problem is addressed by the subject matter with the features of the independent claims. Advantageous embodiments which might be realized in an isolated fashion or in any arbitrary combinations are listed in the dependent claims.

In accordance, the present invention relates to a method for identifying a subject at risk of developing cancer, said method comprising

   (a) determining in a sample of said subject the amount of at least one miRNA selected from the list consisting of miR-19a-3p, let-7g-5p, miR-23a-3p, miR-92a-3p, miR-144-5p, and miR-27a-3p;
   (b) comparing said amount or amounts determined in step (a) to a reference or to references; and
   (c) identifying a subject at risk of developing cancer based on the result of comparing step (b).

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one". In accordance, expressions relating to one item of a plurality, unless otherwise indicated, preferably relate to at least one such item, more preferably a plurality thereof; thus, e.g. identifying "a cell" relates to identifying at least one cell, preferably to identifying a multitude of cells.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

The methods specified herein below, preferably, are in vitro methods. The method steps may, in principle, be performed in any arbitrary sequence deemed suitable by the skilled person, but preferably are performed in the indicated sequence; also, one or more, preferably all, of said steps may be assisted or performed by automated equipment. Moreover, the methods may comprise steps in addition to those explicitly mentioned above.

As used herein, if not otherwise indicated, the term "about" relates to the indicated value with the commonly accepted technical precision in the relevant field, preferably relates to the indicated value $\pm$ 20%, more preferably $\pm$ 10%, most preferably $\pm$ 5%. Further, the term "essentially" indicates that deviations having influence on the indicated result or use are absent, i.e. potential deviations do not cause the indicated result to deviate by more than $\pm$ 20%, more preferably $\pm$ 10%, most preferably $\pm$ 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. In the context of biological sequences referred to herein, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in silico. Thus, as used herein, an Fc or Fab fragment, but also e.g. a single-chain antibody, a bispecific antibody, and a nanobody may be referred to as fragments of an immunoglobulin.

Unless specifically indicated otherwise herein, the compounds specified, in particular the polynucleotides and polypeptides, may be comprised in larger structures, e.g. may be covalently or non-covalently linked to further sequences, carrier molecules, retardants, and other excipients. E.g., the skilled person is aware that miRNAs as referred to herein preferably are produced by cells of a subject from precursors comprising said miRNAs; thus a miRNA may in particular be comprised in a precursor, preferably as specified herein below.

The method for identifying a subject at risk of developing cancer of the present invention, preferably, is an in vitro method. Moreover, it may comprise steps in addition to those explicitly mentioned above. For example, further steps may relate, e.g., to providing a sample of the subject for step a), or providing lifestyle recommendations, adapted medical surveillance, and/or treatment to said subject based on identification step (c). The method for identifying a subject at risk of developing cancer preferably is not a diagnostic method, i.e. preferably does not diagnose existing cancer. Thus, preferably, the method is a method of cancer prediction, preferably at least six months, more preferably at least one year, still more preferably at least two years, even more preferably at least three years, most preferably at least five years before clinical manifestation of cancer. Thus, preferably, the method comprises further step (d1) performing cancer diagnostics on the subject identified to be at risk of developing cancer at least three months, preferably at least six months, more preferably at least one year, still more preferably at least two years, most preferably at least three years after said subject is identified as being at risk of developing cancer. Also preferably, the method comprises a further step (d2) assigning the subject identified to be at risk of developing cancer to colonoscopy at a frequency of at least every three years, preferably at least every two years, more preferably at least yearly, optionally over a period of at least one, preferably at least two, more preferably at least three, years.

The term "subject", as referred to herein, relates to a vertebrate animal, preferably a mammal, more preferably a human. Preferably, the subject does not show a symptom of cancer; symptoms of cancer and diagnostic methods related thereto are known from standard medical textbooks. More preferably, the subject is apparently healthy. Preferably, the subject is known to have an increased risk of developing cancer, more preferably is known to have a genetic disposition for developing cancer and/or has a close first or second-grade relative having developed cancer. More preferably, the subject has an unknown risk of developing cancer. Preferably, the subject is at least 50 years old, more preferably is at least 55 years old. Preferably, the subject is a male subject aged at least 50 or a female subject aged at least 55. It is, however, also envisaged that the subject may be substantially younger, e.g. at least 25 or at least 30 years, in particular in case of a known or suspected predisposition for developing cancer.

The term "sample" refers to a sample of a bodily fluid, preferably a sample of blood, plasma, serum, saliva, sputum, urine, or a sample comprising separated cells or to a sample from a tissue or an organ. Tissue or organ samples may be obtained from any tissue or organ by, e.g., biopsy. Separated cells may be obtained from the body fluids, such as lymph, blood, plasma, serum, liquor and other, or from the tissues or organs by separating techniques such as centrifugation or cell sorting. Preferably, the sample is a tissue or body fluid sample, more preferably the sample is a sample of a body fluid, preferably a blood sample, serum, or plasma sample, most preferably a serum sample. The sample can be obtained from the subject by routine techniques which are well known to the person skilled in the art, e.g., venous or arterial puncture or open biopsy including aspiration of tissue or cellular material from a subject. For those areas which cannot be easily reached via an open biopsy, a surgery and, preferably, minimal invasive surgery can be performed.

The term "cancer", as used herein, relates to a disease of a subject, characterized by uncontrolled growth by a group of body cells ("cancer cells"). This uncontrolled growth may lead to tumor formation and may be accompanied by intrusion into and destruction of surrounding tissue (invasion) and possibly spread of cancer cells to other locations in the body (metastasis). Thus, preferably, the cancer is a solid cancer, a metastasis, and/or a relapse thereof. Preferably, the cancer is a cancer of the gastrointestinal system, more preferably of the colon or the rectum. Thus, preferably, the cancer is colorectal cancer (CRC).

The term "at risk of developing cancer", is in principle understood by the skilled person. Preferably, the term relates to a subject having an increased risk of developing cancer compared to a control population of subjects. Preferably, said risk is increased by a factor of at least 2, more preferably at least 3, still more preferably at least 5, most preferably at least 7, compared to said control population. As indicated herein above, a subject at risk of developing cancer preferably is not known to suffer from cancer at the time the identification is made, more preferably has no symptoms of CRC, most preferably is apparently healthy. Thus, the subject preferably is CRC-free, preferably is cancer -free. In accordance, the subject preferably is first diagnosed with cancer, preferably CRC, at least six months, more preferably at least one year, still more preferably at least two years, most preferably at least three years after being identified as a subject at risk of developing cancer.

The term "determining", as used herein, refers to quantitatively determining an analyte by measuring at least one characteristic feature of the analyte to be determined in a sample. Characteristic features in accordance with the present invention are features which characterize the physical and/or chemical properties including biochemical properties of an analyte. Such properties include, e.g., molecular weight, as e.g. determined by mass spectrometry; nucleic acid sequence, as e.g. determined by sequencing; capability to react with other compounds, in particular hybridization to one or more probe oligonucleotide or primer oligonucleotide(s); capability to elicit a response in a biological read out system (e.g., induction of a reporter gene); and the like. Values for the aforesaid properties may serve as characteristic features and can be determined by techniques well known in the art. For miRNAs, determining may in particular be accomplished by RT-qPCR or sequencing, e.g. next-generation sequencing. Moreover, the value determined may be any value which is derived from the values of the physical and/or chemical properties of an analyte by standard operations, e.g., mathematical calculations such as multiplication, division or logarithmic calculus. Preferably, the value determined for a miRNA is a normalized value as specified herein below.

The term "miRNA" or "microRNA" is understood by the skilled artisan and relates to a short ribonucleic acid (RNA) molecule found in eukaryotic cells and in body fluids of metazoan organisms. The term preferably also encompasses pri-miRNAs and pre-miRNAs of the miRNAs of the present invention. Thus preferably, a miRNA-precursor consists of 25 to several thousand nucleotides, more preferably 40 to 130 nucleotides, even more preferably 50 to 120 nucleotides, or, most preferably 60 to 110 nucleotides. Preferably, a miRNA consists of 5 to 100 nucleotides, more preferably 10 to 50 nucleotides, even more preferably 12 to 40 nucleotides, or, most preferably 18 to 26 nucleotides. Preferably, the miRNAs of the present invention are miRNAs of human origin, i.e. they are encoded in the human genome. Also preferably, the term miRNA relates to the "guide" strand which eventually enters the RNA-induced silencing complex (RISC) as well as to the "passenger" strand complementary thereto. Preferably, the miRNA or miRNAs used in the method specified herein is/are selected from the list consisting of miR-19a-3p, preferably hsa-miR-19a-3p (miRBase (Griffiths-Jones S., NAR 2004 32(Database Issue):D109-D111; Kozomara A, Griffiths-Jones S., NAR 2011 39(Database Issue):D152-D157) Accession No. MIMAT0000073, 5'-ugugcaaaucuaugcaaaacuga-3' (SEQ ID NO:1)); let-7g-5p, preferably hsa-let-7g-5p (miRBase Accession No. MIMAT0000414, 5'-ugagguaguaguuuguacaguu-3' (SEQ ID NO:2)); miR-23a-3p, preferably hsa-miR-23a-3p (miRBase Accession No. MIMAT0000078, 5'-aucacauugccagggauuuucc-3' (SEQ ID NO:3)); miR-92a-3p, preferably hsa-miR-92a-3p (miRBase Accession No. MIMAT0000092, 5'-uauugcacuugucccggccugu-3' (SEQ ID NO:4)); , miR-144-5p, preferably hsa-miR-144-5p (miRBase Accession No. MIMAT0004600, 5'-ggauaucaucauauacuguaag-3' (SEQ ID NO:5)); and miR-27a-3p; preferably hsa-miR-27a-3p (miRBase Accession No. MIMAT0000084, 5'-uucacaguggcuaaguuccgc-3' (SEQ ID NO:6)). Preferably, further miR-21-5p, more preferably hsa-miR-21-5p (miRBase Accession No. MIMAT0000076, 5'-uagcuuaucagacugauguuga-3' (SEQ ID NO:7)) is determined. Also preferably, additionally at least one normalizer miRNA, preferably selected from the list consisting of normalizer miRNAs are miR-93-5p, preferably hsa-miR-93-5p (miRBase Accession No. MIMAT0000093, 5'-caaagugcuguucgugcagguag-3' (SEQ ID NO:8)), miR-1246, preferably hsa-miR-1246 (miRBase Accession No. MIMAT0005898, 5'-aauggauuuuuggagcagg-3' (SEQ ID NO:9)); and miR-223-3p, preferably hsa-miR-223-3p (miRBase Accession No. MIMAT0000280, 5'-ugucaguuugucaaauaccca-3' (SEQ ID NO:10)) is determined.

"Comparing", as used herein, encompasses comparing the amount of a miRNA which is comprised by a sample with an amount of said miRNA in a suitable reference as specified elsewhere herein. More preferably, comparing comprises calculating a score, preferably from a multitude of miRNAs, more preferably as specified herein below. It is to be understood that comparing as referred to herein refers to a comparison of corresponding parameters or values, e.g., an absolute amount of a miRNA is compared to an absolute reference amount; a concentration of a miRNA is compared to a reference concentration; or an intensity signal obtained from the miRNA is compared to the same type of intensity signal in a reference; more preferably, a score is compared to a reference score. The comparison referred to in the methods of the present invention may be carried out manually or computer assisted. For a computer assisted comparison, the value determined may be compared to a value corresponding to a suitable reference which is stored in a database by a computer program. The computer program may further evaluate the result of the comparison by means of an expert system. Accordingly, the result of the identification referred to herein may be automatically provided in a suitable output format.

The terms "reference", "reference value", "reference amount", or "reference score", as used herein, refer to an amount of miRNA or miRNAs or a value derived therefrom which allows assessing if being at risk of developing cancer or not being at risk of developing cancer is to be assumed for the subject from which the sample is derived. A suitable reference may be determined from a reference subject, preferably a population of reference subjects, preceding, simultaneously, or subsequently, with the sample. Preferably, the reference is a pre-determined reference which may be stored e.g. in a database. Also preferably, the reference is a fixed value or range of values for all subjects to be tested.

References can, in principle, be calculated for a group or cohort of subjects as specified herein based on the average or median values for a given miRNA by applying standard methods of statistics. In particular, accuracy of a test such as a method aiming to predict an event, or not, is best described by its receiver-operating characteristics (ROC) (see especially Zweig (1993), Clin. Chem. 39:561). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the sum of number of true-positive and number of false-negative test results. On the x-axis is the false-positive fraction, or 1-specificity, which is defined as the ratio of number of false-positive results to the sum of number of true-negative and number of false-positive results. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. The reference to be used for the methods of the present invention can be generated, preferably, by establishing a ROC for said cohort as described above and deriving a threshold amount therefrom. Dependent on a desired sensitivity and specificity for a diagnostic method, the ROC plot allows deriving suitable thresholds. Preferably, the reference amounts lie within the range of values that represent a sensitivity of at least 75% and a specificity of at least 45%, or a sensitivity of at least 80% and a specificity of at least 40%, or a sensitivity of at least 85% and a specificity of at least 33%, or a sensitivity of at least 90% and a specificity of at least 25%.

Preferably, the reference amount as used herein is derived from samples of a population of subjects for which it is known if their donors were later, e.g. within three years, being afflicted with cancer or not. This reference may be a discrete figure or may be a range of figures. Evidently, the reference may vary between individual species of miRNA. The measuring system therefore, preferably, is calibrated with a sample or with a series of samples comprising known amounts of each specific miRNA. It is understood by the skilled person that in such case the amount of miRNA can preferably be expressed as arbitrary units (AU). Thus, preferably, the amounts of miRNA are determined by comparing the signal obtained from the sample to signals comprised in a calibration curve. More preferably, the amounts of miRNA are obtained as arbitrary units and normalized to the amount(s) of at least one, preferably at least two, more preferably at least three, normalizer miRNAs, preferably as specified herein above. Also more preferably, a score is calculated from at least two miRNAs, preferably as specified herein below. A suitable reference may be determined as specified herein in the Examples. Also, a threshold reference, e.g. a threshold score, can be preferably used as a reference. A reference may, preferably, be derived from a subject or group of subjects being known to have later been afflicted with cancer. A reference amount may, preferably, also be derived from a subject or group of subjects known to later have not been afflicted with cancer. It is to be understood that the aforementioned amounts may vary due to statistics and errors of measurement. A deviation, i.e. a decrease or an increase of the miRNA amounts referred to herein is, preferably, a statistically significant deviation, i.e. a statistically significant decrease or a statistically significant increase.

Step (a) of the method comprises determining in a sample of said subject the amount of at least one miRNA selected from the list consisting of miR-19a-3p, let-7g-5p, miR-23a-3p, miR-92a-3p, miR-144-5p, and miR-27a-3p. Preferably, the method comprises determining the amounts of at least two, preferably at least three, more preferably at least four, still more preferably at least five, even more preferably all six, miRNAs from said list. Also preferably, the method further comprises determining the amount of miR-21-5p; in such case, preferably, all seven aforesaid miRNAs are determined. Preferred miRNAs or combinations thereof to be determined are selected from the list consisting of (i) miR-19a-3p, (ii) miR-144-5p, (iii) miR-19a-3p and miR-144-5p, (iv) miR-19a-3p and let-7g-5p, and (v) miR-21-5p and miR-23a-3p. In view of the above, the amount determined in step (a) preferably is a relative abundance of the miRNA, preferably normalized to a panel of at least two, preferably at least three, normalizer miRNAs, more preferably wherein said normalizer miRNAs are miR-93-5p, miR-1246 and miR-223-3p.

The method further comprises step (b) comparing the amount or amounts determined in step (a) to a reference or to references. The term reference has been specified herein above. As will be appreciated, the method identifying a subject at risk of developing cancer is a predictive method, so a reference typically will be derived from a subject or subjects for whom it is known whether they later developed cancer, preferably within at least one year, preferably at least two years, more preferably at least three years. Thus, the reference and/or the samples used to establish said reference preferably were obtained in the past, preferably the aforesaid time frame in the past. Preferably, the reference is derived from a population of apparently healthy subjects or a population of subjects known not to have developed cancer. Also preferably, the reference is derived from a population of subjects known to have developed cancer. Preferably, step (a) comprises determining the amounts of at least two miRNAs and a score is calculated from said amounts of at least two miRNAs. More preferably, at least three, four, five, or six, miRNAs are determined and a score is calculated from said amounts of miRNAs. Still more preferably, at least seven miRNAs are determined and a score is calculated from said amounts of seven miRNAs. Most preferably, at least seven miRNAs and three normalizer miRNAs are determined and a score is calculated from said amounts of seven miRNAs and three normalizer miRNAs, preferably as specified herein below and/or in the Examples.

Preferably, in step (b) a score is calculated from the amounts of miRNAs determined. Said calculating a score preferably comprises calculating a sum of the amounts of said at least two miRNAs, preferably wherein said amounts are weighted. Preferably for calculating the score the value of each miRNA determined is multiplied with a pre-determined weighting factor w. Preferably, in step (a) the amounts of miR-19a-3p, let-7g-5p, miR-23a-3p, miR-92a-3p, miR-144-5p, miR-21-5p and miR-27a-3p are determined and the score is calculated according to equation (1)

$$score = w1 * miR\text{-}19a\text{-}3p + w2 * let\text{-}7g\text{-}5p + w3 * miR\text{-}23a\text{-}3p + w4 * miR\text{-}92a\text{-}3p + w5 * miR\text{-}144\text{-}5p + w6 * miR\text{-}21\text{-}5p + w7 * miR\text{-}27a\text{-}3p \quad (1);$$

preferably wherein w1= -0.2 ± 0.3; w2 = 0.2 ± 0.3; w3 = 1.7 ± 0.5; w4 = 0.5 ± 0.3; w5 = 0.2 ± 0.2; w6 = -1.7 ± 0.4; and/or w7 = 0.1 ± 0.3. As is understood by the skilled person, in equation (1), as well as in equations (2) and (3) below, the designation of a miRNA stands for a value correlating with its amount in a sample determined as specified herein above, preferably for a normalized amount. Preferably, in the aforesaid equation (1), (i) w1 is -0.2, preferably about -0.20, more preferably -0.20, even more preferably -0.202, most preferably-0.2024; (ii) w2 is 0.2, preferably about 0.24, more preferably 0.24, even more preferably 0.235, most preferably 0.2351; (iii) w3 is 1.7, preferably about 1.7, more preferably 1.66, even more preferably 1.660, most preferably 1.6595; (iv) w4 is 0.5, preferably about 0.48, more preferably 0.48, even more preferably 0.479, most preferably 0.4794; (v) w5 is 0.2, preferably about 0.20, more preferably 0.20, even more preferably 0.200, most preferably 0.2002; (vi) w6 is -1.7, preferably about -1.7, more preferably -1.68, even more preferably-1.677, most preferably -1.6772; and/or (vii) w7 is 0.1, preferably about 0.10, more preferably 0.10, even more preferably 0.101, most preferably 0.1014. More preferably, the score is calculated according to equation (2)

$$score = 0.2 + let\text{-}7g\text{-}5p * 0.2 + miR\text{-}19a\text{-}3p * \text{-}0.2 + miR\text{-}23a\text{-}3p * 1.7 + miR\text{-}92a\text{-}3p * 0.5 + miR\text{-}144\text{-}5p * 0.2 + miR\text{-}21\text{-}5p * \text{-}1.7 + miR\text{-}27a\text{-}3p * 0.1 \quad (2).$$

Most preferably, the score is calculated according to equation (3)

$$score = 0.1899 + let\text{-}7g\text{-}5p * 0.2351 + miR\text{-}19a\text{-}3p * \text{-}0.2024 + miR\text{-}23a\text{-}3p * 1.6595 + miR\text{-}92a\text{-}3p * 0.4794 + miR\text{-}144\text{-}5p * 0.2002 + miR\text{-}21\text{-}5p * \text{-}1.6772 + miR\text{-}27a\text{-}3p * 0.1014 \quad (3).$$

Step (c) of the method comprises identifying a subject at risk of developing cancer based on the result of comparing step (b).

Preferably, the reference is derived from a population of apparently healthy subjects or a population of subjects known not to have developed cancer; in such case, preferably, an amount of miRNA deviating from said reference is indicative of a subject at risk of developing cancer. Thus, in such case, a subject at risk of developing cancer is identified in case (i) an increased amount of let-7g-5p, miR-23a-3p, miR-92a-3p and/or miR-144-5p is determined; and/or (ii) a decreased amount of miR-19a-3p, miR-21-5p, and/or miR-27a-3p is determined. Also in such case, the reference may be the lowest value of the middle quintile of said population for let-7g-5p, miR-23a-3p, miR-92a-3p and/or miR-144-5p; and/or (ii) is the highest value of the middle quintile of said population for miR-19a-3p, miR-21-5p, and/or miR-27a-3p.

Also preferably, the reference in step (b) is derived from a population of subjects known to have developed cancer; in such case, preferably, an amount of miRNA essentially identical to said reference is indicative of a subject at risk of developing cancer.

Also preferably, said reference is a score of the same patient obtained by the same method at least three months (earlier score), preferably at least six months, more preferably at least one year, still more preferably at least two years, before obtaining the score to be evaluated (later score), preferably wherein an increase in the later score compared to said earlier score is indicative of a subject being at risk of developing cancer.

Preferably, the method comprises additional step (d1) recommending to and/or performing on said subject identified to be at risk of developing cancer at least one cancer diagnostic step to be performed at least three months, preferably at least six months, more preferably at least one year, still more preferably at least two years, most preferably at least three years after said subject is identified as being at risk of developing cancer.

Cancer diagnostic steps are known to the skilled person and are selected based on the cancer to be detected. In the case of gastrointestinal cancer, in particular CRC, the diagnostic step is preferably selected from the list consisting of colonoscopy, sigmoidoscopy, contrast enema, computer tomographic colonography, fecal blood test, and/or fecal DNA test. Preferably, recommending a cancer diagnostic test comprises contacting said subject to provide information on the recommendability of said cancer diagnostic test and, optionally, fixing an appointment for performing said cancer diagnostic test. The term performing a cancer diagnostic test is understood by the skilled person.

The cancer diagnostic step may be performed and/or recommended in concurrence with an assumed or identified risk of developing cancer. Thus, the method for identifying a subject at risk of developing cancer may in particular be performed on a subject and/or at a point in time where risk assessment is deemed appropriate. Thus, the method for identifying a subject at risk of developing cancer is preferably performed on a sample of a subject at least 50 years old, more preferably at least 55 years old. Preferably, the subject is a biological female and the method is first performed at an age of at least 55; also preferably, the subject is a biological male and the method is first performed at an age of at least 50. It is however, also envisaged to perform the method on subjects at a younger age e.g. 30 years or 35 years, in particular in subjects with a known risk of developing cancer, such as CRC. Thus, the method may in particular be performed on a subject with a relative known to have developed cancer at an age at least ten years before the age at which said relative developed cancer.

Preferably, step (d1) further comprises adjusting the timing of cancer diagnostic steps in dependence on whether the subject was identified to be at risk of developing cancer or not. Thus, for a subject identified to be not at risk of developing cancer, the first cancer diagnostic step may be recommended and/or performed at least five, more preferably at least ten years after said identification, and intervals of said cancer diagnostic steps may be in the same time range. In contrast, for a subject identified to be not at risk of developing cancer, the first cancer diagnostic steps may be recommended and/or performed within at most four, preferably at most three, years after said identification and intervals of said cancer diagnostic steps may be in the same time frame. Thus, the method preferably comprises further step (d2) assigning the subject identified to be at risk of developing cancer to colonoscopy at a frequency of at least every three years, preferably at least every two years, more preferably at least yearly, optionally over a period of at least one, preferably at least two, more preferably at least three, years.

The method may comprise further step (d3) providing lifestyle recommendations and/or adapted medical surveillance to said subject. Lifestyle recommendations to mitigate cancer risk are known in the art. Thus, providing lifestyle recommendations may comprise contacting the subject, providing to and optionally discussing with the subject information about benefits of lifestyle changes. Adaptations of medical surveillance include in particular scheduling and performing follow-up appointments with a medical practitioner at an increased frequency, e.g. every 3 months to 2 years. Thus, as used herein, the term "close monitoring", as used herein, relates to performing medical surveillance as specified herein above, while "loose monitoring" relates to performing medical surveillance at a lower frequency, e.g. every four to five years. Thus, close monitoring preferably comprises performing cancer diagnostics on the subject identified to be at risk of developing cancer at least every three months, preferably at least every six months, more preferably at least once a year, preferably over a period of at least one year, more preferably at least two years, most preferably at least three years; and also preferably, loose monitoring comprises performing cancer diagnostics on the subject identified to be not at risk of developing cancer at most every three years, preferably at most every five years, more preferably comprises not performing cancer diagnostics on the subject identified to be not at risk of developing cancer for at least three years after identifying the subject to be not at risk of developing cancer.

Advantageously, it was found in the work underlying the present invention that the biomarkers as specified can indicate an increased risk of developing cancer, with a prediction time frame of several years before the actual onset of cancer.

The definitions made above apply mutatis mutandis to the following. Additional definitions and explanations made further below also apply for all embodiments described in this specification mutatis mutandis.

The present invention further relates to a method of identifying a subject not at risk of developing CRC comprising steps a) and b) of the method for identifying a subject at risk of developing cancer, and step c1) identifying a subject not at risk of developing CRC based on the result of comparing step (b).

The present invention also relates to a method for monitoring a patient for development of cancer comprising steps a) and b) of the method for identifying a subject at risk of developing cancer, and step (c2) identifying a subject being at risk of developing cancer based on the result of step (b) and performing close monitoring of said subject; and/or (c3) identifying a subject being not at risk of developing cancer based on the result of step (b) and performing loose monitoring of said subject.

The present invention also relates to an integrated diagnostic method comprising (A) identifying a subject at risk of developing cancer according to the method for identifying a subject at risk of developing cancer, and (B) performing cancer diagnostics on the subject identified to be at risk of developing cancer at least three months, preferably at least six months, more preferably at least one year, still more preferably at least two years, most preferably at least three years after said subject is identified as being at risk of developing cancer.

Further, the present invention relates to a method of diagnosing and treating cancer comprising (A) identifying a subject at risk of developing cancer the method for identifying a subject at risk of developing cancer, (B) performing cancer diagnostics on the subject identified to be at risk of developing cancer at least three months, preferably at least six months, more preferably at least one year, still more preferably at least two years, most preferably at least three years after said subject is identified as being at risk of developing cancer, thereby identifying a subject suffering from cancer; and (C) treating cancer in a subject identified to suffer from cancer in step (C).

The terms "treating" and "treatment" refer to an amelioration of the diseases or disorders referred to herein or the symptoms accompanied therewith to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20% at least 50%, at least 60%, at least 70%, at least 80%, or at least 90% of the subjects of a given cohort or population. Preferably, treating cancer is reducing tumor burden in a subject. As will be understood by the skilled person, methods and effectiveness of treatment of e.g. cancer is dependent on a variety of factors including, e.g. cancer stage and cancer type.

Preferably, treating comprises surgery, chemotherapy, radiotherapy, targeted therapy, and/or immunotherapy.

The terms "radiation therapy" and "radiotherapy" are known to the skilled artisan. The terms relate to the use of ionizing radiation to treat or control cancer. The skilled person also knows the term "surgery", relating to invasive measures for treating cancer, in particular excision of tumor tissue.

As used herein, the term "chemotherapy" relates to treatment of a subject with an antineoplastic drug. Preferably, chemotherapy is a treatment including alkylating agents (e.g. cyclophosphamide), platinum (e.g. carboplatin), anthracyclines (e.g. doxorubicin, epirubicin, idarubicin, or daunorubicin) and topoisomerase II inhibitors (e.g. etoposide, irinotecan, topotecan, camptothecin, or VP16), anaplastic lymphoma kinase (ALK)-inhibitors (e.g. Crizotinib or AP26130), aurora kinase inhibitors (e.g. N-[4-[4-(4-Methylpiperazin-1-yl)-6-[(5-methyl-1H-pyrazol-3-yl)amino]pyrimidin-2-yl]sulfanylphenyl]cyclopropanecarboxamide (VX-680)), antiangiogenic agents (e.g. Bevacizumab), or Iodine131-1-(3-iodobenzyl)guanidine (therapeutic metaiodobenzylguanidine), histone deacetylase (HDAC) inhibitors, alone or any suitable combination thereof. It is to be understood that chemotherapy, preferably, relates to a complete cycle of treatment, i.e. a series of several (e.g. four, six, or eight) doses of antineoplastic drug or drugs applied to a subject separated by several days or weeks without such application.

The term "targeted therapy", as used herein, relates to application to a patient of a chemical substance known to block growth of cancer cells by interfering with specific molecules known to be necessary for tumorigenesis or cancer or cancer cell growth. Examples known to the skilled artisan are small molecules like, e.g. PARP-inhibitors (e.g. Iniparib), or monoclonal antibodies like, e.g., Trastuzumab.

The term "immunotherapy" as used herein relates to the treatment of cancer by modulation of the immune response of a subject, e.g. as cell based immunotherapy. Said modulation may be inducing, enhancing, or suppressing said immune response. The term "cell based immunotherapy" relates to a cancer therapy comprising application of immune cells, e.g. T-cells, preferably tumor-specific NK cells, to a subject.

The tem "preventing" refers to retaining health with respect to the diseases or disorders referred to herein for a certain period of time in a subject. It will be understood that the said period of time may be dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The present invention also relates to a kit comprising means for determining the amounts of at least six, more preferably all seven, miRNAs selected from the list consisting of miR-19a-3p, let-7g-5p, miR-23a-3p, miR-92a-3p, miR-144-5p, miR-21-5p and miR-27a-3p, preferably comprised in a housing.

The term "kit", as used herein, refers to a collection of the aforementioned compounds, means or reagents which may or may not be packaged together. The components of the kit may be comprised by separate vials (i.e. as a kit of separate parts) or provided in a single vial, e.g. as a composition as specified herein above. The housing of the kit in an embodiment allows translocation of the compounds of the kit, in particular common translocation; thus, the housing may in particular be a transportable container comprising all specified components. Moreover, it is to be understood that the kit of the present invention may be used for practicing the methods referred to herein above. It is, in an embodiment, envisaged that all components are provided in a ready-to-use manner for practicing the methods referred to above. Further, the kit, in an embodiment, contains instructions for carrying out said methods. The instructions can be provided by a user's manual in paper- or electronic form. For example, the manual may comprise instructions for interpreting the results obtained when carrying out the aforementioned methods using the kit. Preferably, the kit comprises further compounds, such as a reaction buffer, a hybridization solution, a lysis buffer, and the like. Also preferably, the kit further comprises means for determining at least one, preferably at least two, more preferably all three, miRNAs selected from the group consisting of miR-93-5p, miR-1246, and miR-223-3p. Preferably, the kit is adapted for use in a method of the present invention, more preferably is adapted to comprise all reagents required to perform said method or methods.

The term "means for determining a miRNA" is understood by the skilled person to relate to a means adapted for specifically determining said miRNA, preferably in a sample as specified herein above. Thus, a means for determining may in particular be a probe oligonucleotide and/or a primer oligonucleotide, preferably specifically hybridizing to said miRNA.

The present invention also relates to a device comprising an evaluation unit with a processor, and, preferably tangibly embedded, instructions which, when performed on the processor, cause the device to perform at least step (b) of the method for identifying a subject at risk of developing cancer.

The term "device", as used herein, relates to a system of means comprising at least the aforementioned means operatively linked to each other as to allow the determination. Typical means for carrying out the determination are disclosed herein above. An evaluation unit is any means capable of providing the analysis as specified; preferably, the evaluation unit is or comprises a data processing means, such as a microprocessor, a handheld device such as a mobile phone, or a computer. How to link the means in an operating manner will depend on the type of means included into the device. Preferably, the means are comprised by a single device. Preferably, the instructions and interpretations are comprised in an executable program code comprised in the device, such that, as a result of determination, an identification of a subject at risk of developing cancer or not may be output to a user. Typical devices are those which can be applied without the particular knowledge of a specialized technician, e.g., electronic devices which merely require loading data, e.g. results of step (a) of the method. The output may be given as output of raw data which need interpretation by a technician. In an embodiment, the output of the device is, however, processed, i.e. evaluated, raw data, the interpretation of which does not require a technician. Preferably, the the device further comprises a memory unit, preferably comprising a database comprising at least one reference value for a miRNA or a score derived therefrom. Also preferably, the device further comprises a data input device, allowing input of miRNA values determined in step (a) of the method.

The present invention further relates to a system comprising a device as specified herein above and an measuring unit adapted for measuring the amount of at least one miRNA specified in the method for identifying a subject at risk of developing cancer.

The term "system", as used herein, relates to a system of means comprising at least the aforementioned means operatively linked to each other as to allow the result of the detection to be obtained. Preferred means for determining miRNAs are disclosed above in connection with the methods of the invention. How to link the means in an operating manner will depend on the type of means included into the system. In an embodiment, the means of the system are comprised by a single housing.

Preferably, the measuring unit comprises a receptacle for a sample. The receptacle may directly contact the sample, or may be a receptacle for a further means receiving the sample, wherein the further means may be e.g. a multi-well plate, to which a sample or a multiplicity of samples may be applied. Moreover, the sample treatment unit preferably comprises a reservoir connected to a dosing means, e.g. a tubing connected to a pump, preferably comprising a lysis buffer. Preferably, the measuring unit comprises at least one detector compound, e.g. in a dried form or in a reservoir connected to a dosing means, e.g. a tubing connected to a pump.

Preferably, the measuring unit further comprises a detection unit for detecting a miRNA. Means suitable as a detection unit according to the present invention are known to the skilled person. Typical means for determining miRNAs, and means for carrying out the determination are disclosed herein above. As is understood by the skilled person, means for determining miRNAs include means capable for determining an amount of a miRNAs, such as a quantitative PCR unit or an MS unit, or means for determining miRNAs activity, such as an optical unit detecting a signal of a reporter gene assay. Further typical systems comprise the detection units (e.g., biosensors, arrays, solid supports coupled to ligands specifically recognizing the miRNA, Plasmon surface resonance devices, NMR spectrometers, mass-spectrometers etc.).

Furthermore, the present invention relates to a method of measuring biomarkers comprising (a) determining in a sample the amount of at least one miRNA selected from the list consisting of miR-19a-3p, let-7g-5p, miR-23a-3p, miR-92a-3p, miR-144-5p, and miR-27a-3p; and (b) optionally calculating a score from said at least one miRNA.

The method of measuring biomarkers of the present invention is an in vitro method. Moreover, it may comprise steps in addition to those explicitly mentioned above. For example, further steps may relate, e.g., to determining further miRNAs or other biomarkers in step a), or outputting the result in step b). Moreover, one or more of said steps may be assisted or performed by automated equipment.

In view of the above, the following embodiments are particularly envisaged:

Embodiment 1: A method for identifying a subject at risk of developing cancer, said method comprising

(a) determining in a sample of said subject the amount of at least one miRNA selected from the list consisting of miR-19a-3p, let-7g-5p, miR-23a-3p, miR-92a-3p, miR-144-5p, and miR-27a-3p;
(b) comparing said amount or amounts determined in step (a) to a reference or to references; and
(c) identifying a subject at risk of developing cancer based on the result of comparing step (b).

Embodiment 2: The method of embodiment 1, wherein said cancer is colorectal cancer (CRC).

Embodiment 3: The method of embodiment 1 or 2, wherein said subject has no symptoms of CRC, preferably is apparently healthy.

Embodiment 4: The method of any one of embodiments 1 to 3, wherein said subject is CRC-free, preferably is cancer-free.

Embodiment 5: The method of any one of embodiments 1 to 4, wherein said subject is first diagnosed with cancer at least six months, preferably at least one year, more preferably at least two years, most preferably at least three years after being identified as a subject at risk of developing cancer.

Embodiment 6: The method of any one of embodiments 1 to 5, wherein said method comprises determining the amounts of at least two, preferably at least three, more preferably at least four, still more preferably at least five, even more preferably all six, miRNAs miRNAs from said list.

Embodiment 7: The method of any one of embodiments 1 to 6, wherein said at least one miRNA is miR-19a-3p or miR-144-5p.

Embodiment 8: The method of any one of embodiments 1 to 7, wherein at least two miRNAs are determined and wherein said at least two miRNAs are (i) miR-19a-3p and miR-144-5p or are (ii) miR-19a-3p and let-7g-5p.

Embodiment 9: The method of any one of embodiments 1 to 8, wherein said method further comprises determining the amount of miR-21-5p in step (a) and, preferably, comparing said amount to a reference in step (b).

Embodiment 10: The method of embodiment 9, wherein at least two miRNAs are determined and wherein said at least two miRNAs are miR-21-5p and miR-23a-3p.

Embodiment 11: The method of any one of embodiments 1 to 10, wherein at least three miRNAs are determined.

Embodiment 12: The method of any one of embodiments 1 to 11, wherein six miRNAs are determined.

Embodiment 13: The method of any one of embodiments 9 to 11, wherein seven miRNAs are determined.

Embodiment 14: The method of any one of embodiments 1 to 13, wherein the amount determined in step (a) is a relative abundance of the miRNA, preferably compared to a panel of at least two, preferably at least three, normalizer miRNAs, more preferably wherein said normalizer miRNAs are miR-93-5p, miR-1246 and miR-223-3p.

Embodiment 15: The method of any one of embodiments 1 to 14, wherein the reference in step (b) is derived from a population of apparently healthy subjects or a population of subjects known not to have developed cancer; preferably wherein an amount of miRNA deviating from said reference is indicative of a subject at risk of developing cancer.

Embodiment 16: The method of embodiment 15, wherein (i) an increased amount of let-7g-5p, miR-23a-3p, miR-92a-3p and/or miR-144-5p; and/or (ii) a decreased amount of miR-19a-3p, miR-21-5p, and/or miR-27a-3p; is indicative of a subject at risk of developing cancer.

Embodiment 17: The method of embodiment 15 or 16, wherein the reference is the lowest value of the upper quintile of said population for let-7g-5p, miR-23a-3p, miR-92a-3p and/or miR-144-5p; and/or (ii) is the highest value of the lower quintile of said population for miR-19a-3p, miR-21-5p, and/or miR-27a-3p.

Embodiment 18: The method of any one of embodiments 1 to 14, wherein the reference in step (b) is derived from a population of subjects known to have developed cancer; preferably wherein an amount of miRNA essentially identical to said reference is indicative of a subject at risk of developing cancer.

Embodiment 19: The method of any one of embodiments 1 to 18, wherein in step (a) the amounts of at least two miRNAs are determined and wherein in step (b) a score is calculated from said amounts of at least two miRNAs.

Embodiment 20: The method of embodiment 19, wherein said score is calculated as a sum of the amounts of said at least two miRNAs, preferably wherein said amounts are weighted.

Embodiment 21: The method of embodiment 19 or 20, wherein for calculating said score the value of each miRNA determined is multiplied with a pre-determined weighting factor w.

Embodiment 22: The method of any one of embodiments 19 to 21, wherein the amounts of miR-19a-3p, let-7g-5p, miR-23a-3p, miR-92a-3p, miR-144-5p, miR-21-5p and miR-27a-3p are determined and wherein said score is calculated according to equation (1)

$$\text{score} = w1*\text{miR-19a-3p} + w2*\text{let-7g-5p} + w3*\text{miR-23a-3p} + w4*\text{miR-92a-3p} + w5*\text{miR-144-5p} + w6*\text{miR-21-5p} + w7*\text{miR-27a-3p} \quad (1);$$

preferably wherein w1= -0.2 ± 0.3; w2 = 0.2 ± 0.3; w3 = 1.7 ± 0.5; w4 = 0.5 ± 0.3; w5 = 0.2 ± 0.2; w6 = -1.7 ± 0.4; and/or w7 = 0.1 ± 0.3.

Embodiment 23: The method of any one of embodiments 19 to 22, wherein

(i) w1 is -0.2, preferably about -0.20, more preferably -0.20, even more preferably -0.202, most preferably -0.2024;
(ii) w2 is 0.2, preferably about 0.24, more preferably 0.24, even more preferably 0.235, most preferably 0.2351;
(iii) w3 is 1.7, preferably about 1.7, more preferably 1.66, even more preferably 1.660, most preferably 1.6595;
(iv) w4 is 0.5, preferably about 0.48, more preferably 0.48, even more preferably 0.479, most preferably 0.4794;
(v) w5 is 0.2, preferably about 0.20, more preferably 0.20, even more preferably 0.200, most preferably 0.2002;
(vi) w6 is -1.7, preferably about -1.7, more preferably -1.68, even more preferably -1.677, most preferably -1.6772; and/or
(vii) w7 is 0.1, preferably about 0.10, more preferably 0.10, even more preferably 0.101, most preferably 0.1014.

Embodiment 24: The method of any one of embodiments 19 to 23, wherein said score is calculated according to equation (2)

$$\text{score} = 0.2 + \text{let-7g-5p}*0.2 + \text{miR-19a-3p}*{-0.2} + \text{miR-23a-3p}*1.7 + \text{miR-92a-3p}*0.5 + \text{miR-144-5p}*0.2 + \text{miR-21-5p}*{-1.7} + \text{miR-27a-3p}*0.1 \quad (2).$$

Embodiment 25: The method of any one of embodiments 19 to 24, wherein said score is calculated according to equation (3)

$$\text{score} = 0.1899 + \text{let-7g-5p}*0.2351 + \text{miR-19a-3p}*{-0.2024} + \text{miR-23a-3p}*1.6595 + \text{miR-92a-3p}*0.4794 + \text{miR-144-5p}*0.2002 + \text{miR-21-5p}*{-1.6772} + \text{miR-27a-3p}*0.1014 \quad (3).$$

Embodiment 26: The method of any one of embodiments 19 to 25, wherein said reference in step (b) is a reference score derived from a population of apparently heathy subjects or a population of subjects known not to develop cancer, wherein said reference is the middle quintile of scores in said population.

Embodiment 27: The method of any one of embodiments 19 to 26, wherein said reference is a score of the same patient obtained by the same method at least three months (earlier score), preferably at least six months, more preferably at least one year, still more preferably at least two years, before obtaining the score to be evaluated (later score), preferably wherein an increase in the later score compared to said earlier score is indicative of a subject being at risk of developing cancer.

Embodiment 28: The method of any one of embodiments 1 to 27, wherein said method comprises further step (d1) recommending to and/or performing on said subject identified to be at risk of developing cancer at least one cancer diagnostic step to be performed at least three months, preferably at least six months, more preferably at least one year, still more preferably at least two years, most preferably at least three years after said subject is identified as being at risk of developing cancer

Embodiment 29: The method of embodiment 28, wherein said performing cancer diagnostics comprises at least one diagnostic method selected from colonoscopy, sigmoidoscopy, contrast enema, computer tomographic colonography, fecal blood test, and/or fecal DNA test, preferably wherein said performing cancer diagnostics comprises colonoscopy and/or sigmoidoscopy.

Embodiment 30: The method of any one of embodiments 1 to 29, wherein said method comprises further step (d2) assigning the subject identified to be at risk of developing cancer to colonoscopy at a frequency of at least every three years, preferably at least every two years, more preferably at least yearly, optionally over a period of at least one, preferably at least two, more preferably at least three, years.

Embodiment 31: The method of any one of embodiments 1 to 30, wherein said method comprises further step (d3) providing lifestyle recommendations and/or adapted medical surveillance to said subject.

Embodiment 32: A method of identifying a subject not at risk of developing CRC comprising steps a) and b) of the method according to any one of embodiments 1 to 31, and step c1) identifying a subject not at risk of developing CRC based on the result of comparing step (b).

Embodiment 33: A method for monitoring a patient for development of cancer comprising steps a) and b) of the method according to any one of embodiments 1 to 31, and step (c2) identifying a subject being at risk of developing cancer based on the result of step (b) and performing close monitoring of said subject; and/or (c3) identifying a subject being not at risk of developing cancer based on the result of step (b) and performing loose monitoring of said subject.

Embodiment 34: The method of embodiment 33, wherein close monitoring comprises performing cancer diagnostics on the subject identified to be at risk of developing cancer at least every three months, preferably at least every six months, more preferably at least once a year, preferably over a period of at least one year, more preferably at least two years, most preferably at least three years.

Embodiment 35: The method of embodiment 33 or 34, wherein loose monitoring comprises performing cancer diagnostics on the subject identified to be not at risk of developing cancer at most every three years, preferably at most every five years, more preferably comprises not performing cancer diagnostics on the subject identified to be not at risk of developing cancer for at least three years after identifying the subject to be not at risk of developing cancer.

Embodiment 36: An integrated diagnostic method comprising (A) identifying a subject at risk of developing cancer according to the method according to any one of embodiments 1 to 31, and (B) performing cancer diagnostics on the subject identified to be at risk of developing cancer at least three months, preferably at least six months, more preferably at least one year, still more preferably at least two years, most preferably at least three years after said subject is identified as being at risk of developing cancer.

Embodiment 37: A method of diagnosing and treating cancer comprising (A) identifying a subject at risk of developing cancer the method according to any one of embodiments 1 to 31, (B) performing cancer diagnostics on the subject identified to be at risk of developing cancer at least three months, preferably at least six months, more preferably at least one year, still more preferably at least two years, most preferably at least three years after said subject is identified as being at risk of developing cancer, thereby identifying a subject suffering from cancer; and (C) treating cancer in a subject identified to suffer from cancer in step (C).

Embodiment 38: A kit comprising means for determining the amounts of at least six, more preferably all seven, miRNAs selected from the list consisting of miR-19a-3p, let-7g-5p, miR-23a-3p, miR-92a-3p, miR-144-5p, miR-21-5p and miR-27a-3p, preferably comprised in a housing.

Embodiment 39: The kit of embodiment 38, further comprising means for determining at least one, preferably at least two, more preferably all three, miRNAs selected from the group consisting of miR-93-5p, miR-1246, and miR-223-3p.

Embodiment 40: A device comprising an evaluation unit with a processor, and, preferably tangibly embedded, instructions which, when performed on the processor, cause the device to perform at least step (b) of the method according to any one of embodiments 1 to 31.

Embodiment 41: A system comprising the a device according to embodiment 39 and an measuring unit adapted for measuring the amount of at least one miRNA specified in the method according to any one of embodiments 1 to 31.

Embodiment 42: A method of measuring biomarkers comprising

(a) determining in a sample the amount of at least one miRNA selected from the list consisting of miR-19a-3p, let-7g-5p, miR-23a-3p, miR-92a-3p, miR-144-5p, and miR-27a-3p;
(b) optionally calculating a score from said at least one miRNA.

All references cited in this specification are herewith incorporated by reference with respect to their entire disclosure content and the disclosure content specifically mentioned in this specification.

Figure Legends

Figure 1. Flow diagram for selection of prospective set participants; CRC, colorectal cancer.

Figure 2. Receiver operating characteristic curves for CRC risk prediction in prospective set participants with all scores available ($N_{cases/controls}$ = 181/174) according to microRNA risk score (miR-score), polygenic risk score (PRS) and environmental risk score (ERS).

The following Examples shall merely illustrate the invention. They shall not be construed, whatsoever, to limit the scope of the invention.

Example 1: Methods

1.1 Study design and populations

We adopted a two-step approach with a marker discovery and a marker validation phase. For the marker discovery phase, we used pre-treatment plasma samples from patients with newly diagnosed CRC and from controls without CRC recruited between 2016 and 2019 in the context of the GEKKO study. Briefly, the study includes two arms. In arm A, participants who underwent colonoscopy screening in medical practices and clinics in and around Heidelberg, Germany were recruited. In arm B, patients diagnosed with gastrointestinal, lung or breast cancer at the University Hospital Heidelberg were recruited. Participants filled out questionnaires (regarding socio-demographic characteristics, lifestyle factors) and provided biospecimens (blood, saliva, urine, stool and breath condensate) which were processed in a central laboratory and stored in a biobank at -80°C within 4 hours. Colonoscopy reports (arm A) and hospital discharge letters (arm B) were provided by the treating physicians. The study was approved by the ethics committees of the Medical Faculties of the University Heidelberg (S-392/2015), the Eberhard Karls University and the University Hospital Tübingen (876/2017BO2), the physicians' boards of Baden-Württemberg (B-F-2016-034) and of Rhineland Palatinate (2018-13334_5). All participants provided written informed consent.

For the marker validation phase, we used serum samples from the ESTHER study, an ongoing population-based cohort study conducted in Saarland, Germany. In total, 9949 participants aged 50-75 years were recruited between July 2000 and December 2002 by their general practitioners in the context of a general health screening examination, and they have been regularly followed-up thereafter. Information on socio-demographic characteristics, lifestyle factors, and health status at baseline was obtained by standardized self-administered questionnaires. In addition, biological samples (blood, stool, and urine) were collected and stored at -80°C until analysis. Prevalent and incident cancers were determined by record linkage with data from the Saarland Cancer Registry. The study was approved by the ethics committees of the Medical Faculty of the University of Heidelberg and of the state medical board of Saarland, Germany. All participants provided written informed consent.

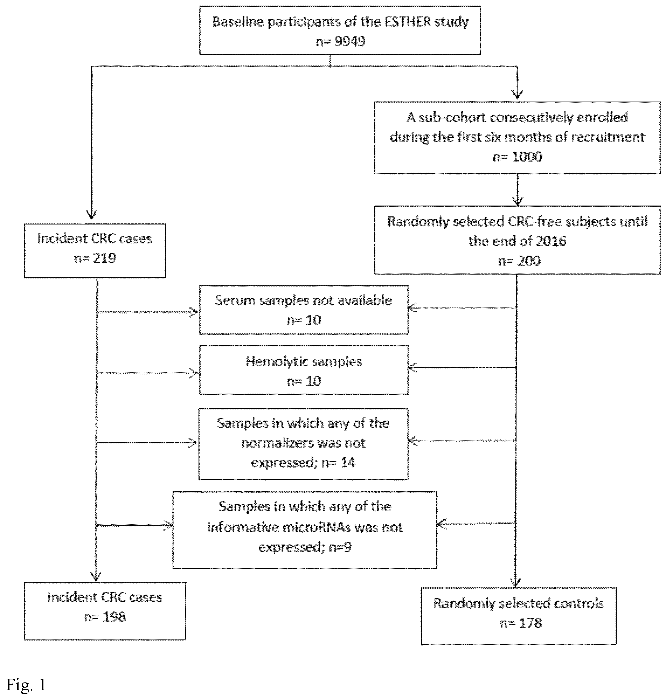

In the marker discovery phase, we identified potential miRNA candidates using genome-wide profiling with NGS in plasma samples from a retrospective set (discovery set) of the GEKKO study that included 20 newly diagnosed CRC cases (from GEKKO arm B) and 20 controls free of colorectal neoplasm (from GEKKO arm A) matched by age and sex. Further candidates were identified through a literature review. MiRNA candidates obtained from the two sources were then measured in a case-cohort approach in baseline serum samples from incident CRC cases identified within 14 years of follow-up and randomly selected controls in the ESTHER study. More specifically, we used qPCR to profile miRNAs in serum collected at baseline from 198 participants with incident CRC and 178 randomly selected participants without diagnosis of CRC until the end of 2016 from participants enrolled during the first 6 months of recruitment (Figure 1).

1.2 MiRNA discovery by next-generation sequencing (NGS)

Plasma samples from the discovery set (GEKKO study) were thawed on ice and then centrifuged at 3000 × g for 5 min at 4°C. RNA was isolated using an miRNeasy Plasma/Serum Kit (QIAGEN) as per the manufacturer's instructions. Five μl total RNA was converted into miRNA NGS libraries using the QIAseq miRNA Library Kit (QIAGEN) as per the manufacturer's instructions. Adapters containing unique molecular identifiers were ligated to the RNA before conversion to cDNA. After PCR (22 cycles), the samples were purified. Library preparation quality control was performed using either Bioanalyzer 2100 (Agilent, Santa Clara, California, United States) or TapeStation 4200 (Agilent). The libraries were pooled in equimolar ratios and sequenced on a NextSeq 500 sequencing instrument as per manufacturer's instructions. FASTQ files were generated using the bcl2fastq software (version 2.2.0, Illumina Inc.) and checked using the FastQC tool. The reads were mapped to the GRCh37 reference genome using Bowtie2 (version 2.2.2). Reads were normalized using TMM method. Differential expression analysis was performed using edgeR (version 3.12.1).

1.3 Selecting miRNA candidates for validation

For the 912 miRNAs profiled using NGS, the raw data was normalized to TMM values and an exact test on the negative binomial distribution was applied to discover differentially expressed miRNAs between CRC cases and controls. The P values were adjusted to the number of comparisons using the Benjamini-Hochberg method, which yields a FDR to control Type I error. 34 miRNAs were found to be differentially expressed between CRC patients and controls. Candidates were then selected using the following inclusion criteria: 1. FDR <0.05; 2. TMM >10 in either case or control group and; 3. |log2FC| >1. Twenty miRNAs meeting these criteria were selected as NGS candidates.

To select additional candidates from the literature, we searched PubMed for publications until 25th April 2019 reporting plasma and serum miRNAs with externally or internally validated AUC values to discriminate CRC patients from controls. Among the 64 literature candidates, we identified 8 miRNAs (miR-202-3p, miR-4669, miR-422a, miR-1290, miR-18b-5p, miR-17-3p, miR-31-5p and miR-204-5p) also detected by NGS, but not meeting the criterion for expression levels (TMM > 10 in at least one of the groups). We excluded these miRNAs, after which 56 literature candidates were further taken into consideration. Finally, we selected 21 miRNAs (individual or combined as a panel) with the highest AUCs and not overlapping with the NGS candidates as literature candidates. In total, 41 miRNA candidates were selected for qPCR profiling in the prospective set.

1.4 MiRNA validation by quantitative real-time PCR (qPCR)

Serum samples from the prospective cohort (ESTHER study) were thawed on ice and centrifuged at 3000 $\times$ g for 5 min at 4°C. Total RNA was extracted from the samples using miRCURY™ RNA Isolation Kit - Biofluids (QIAGEN, Germany) as per manufacturer's instructions. Two $\mu$l RNA was reversely transcribed in ten $\mu$l reactions using the miRCURY LNA RT Kit (QIAGEN). cDNA was diluted 50x and assayed in ten ul PCR reactions according to the protocol for miRCURY LNA miRNA PCR. In a pre-analytical phase, spike-in controls UniSp2, UniSp4, and UniSp6 were added to control for RNA extraction efficiency and possible cDNA synthesis inhibitors. Hemolysis was assessed by determining the levels of miR-451 and miR-23a via qPCR. miR-451 is expressed in red blood cells and miR-23a is relatively stable in serum and not affected by hemolysis. A Cq ratio between miR-23a and miR-451 higher than 7.0 was considered indicative of sample hemolysis. Corresponding samples were excluded from further analysis.

For samples meeting the quality control criteria, each miRNA was assayed once on a custom panel using miRCURY LNA SYBR Green master mix. The primers for miRNAs are listed in Supplementary Table 4. Negative controls excluding template from the reverse transcription reaction were performed and profiled like the samples. The amplification was performed in 384 well plates on a LightCyclerG 480 Real-Time PCR System (Roche). The amplification curves were analyzed using the Roche LC software (version 1.5.0), both for the determination of Cq (Cq was calculated as the 2nd derivative) and for melting curve analysis. The amplification efficiency was calculated using algorithms similar to the LinReg software. All assays were inspected for distinct melting curves and the melting temperature was checked to be within known specifications for the assay. Furthermore, assays within 5 Cq of the negative control or Cq>37 were excluded from further analysis. Detectable miRNAs were those with a Cq value <40.

All laboratory analyses were performed blinded with respect to disease status or findings at colonoscopy.

1.5 Statistical analysis

1.5.1 qPCR data normalization and development of a microRNA risk score (miR-score)

Using the NormFinder method, a combination of three miRNAs (miR-93-5p, miR-1246 and miR-223-3p) exhibiting the highest stability across all samples (stability value= $1.04 \times 10^{-3}$) was identified as normalizers. Samples with missing values for any of the normalizers were excluded from further analysis (n= 14). After selection of three normalizers, 38 miRNAs remained as candidates for evaluation in the validation phase. Of the 38 miRNAs, seven were detectable in at least 99% of the included samples and were identified as informative miRNAs. Samples with missing values for any of the informative miRNAs were excluded from further analysis (n= 9) and the remaining were included in the prospective set. The data was normalized to the average Cq value of the normalizers. The informative miRNAs were then utilized as a panel for fitting a logistic regression model on CRC risk, based on the prospective set. A miR-score was calculated for each participant by summing the observed expression levels of the seven miRNAs weighted by the estimated regression coefficients in the prospective set.

1.5.2 Environmental risk score (ERS)

For the prospective set, information on environmental risk factors, including sociodemographic and lifestyle factors was extracted from participants' questionnaires administered at baseline. Considering the availability of variables, we applied a modified version of a previously derived ERS to predict the presence of CRC in our prospective set. The previously derived ERS used a variable 'waist circumference' which was not available in the ESTHER study and hence we replaced it with 'body mass index'. Both variables are positively correlated and commonly used to assess weight-related health risks. The ERS for each participant was built by summing up the score points for age (0 for <55 years, 1 for 55 to <60 years, 2 for 60 to <65 years, 3 for 65 to <70 years, or 4 for ≥70 years), sex (0 for female or 1 for male), first-degree relative with CRC (1 for ≥1 relative or 0 for other), body mass index (0 for < 25, 1 for 25 to <30, or 2 for ≥30 kg/m2), and cigarette smoking (0 for 0 pack-years, 2 for >0 to <30 pack-years, or 4 for ≥30 pack-years). Missing data for first-degree relative with CRC (n= 5, 1.3%), and cigarette smoking pack-years (n= 31, 8.1%) were imputed by chained equations.

1.5.3 Polygenic risk score (PRS)

Extracted DNA from blood cell collected at baseline was genotyped using the Illumina OncoArray BeadChip (for 82% of the participants) and Global Screening Array (for 18% of the participants). Quality control of the genotype data was performed following a standardized protocol. Missing genotypes (~40 million SNPs) were imputed using Haplotype Reference Consortium (version r1.1.2016) as reference panel within the Michigan Imputation Server. PLINK (version 1.9) was used to extract SNPs for the required region of interest. For building the PRS, a very recently reported set of 140 SNPs that were identified to be associated with a higher risk of CRC were considered. The PRS for each participant was calculated as a weighted sum of risk alleles using weights reported by Thomas et al. (2020).

1.5.4 Associations of the risk scores with CRC risk

To use the scores as risk stratification tools, the participants were stratified into five risk categories using quintile thresholds of the scores in the controls. Based on logistic regression models, ORs along with 95% CIs were estimated for CRC incidence taking the middle quintile as the reference group. Models were calculated first without adjusting for any confounders (Model 1); then additionally adjusting for age and sex (Model 2).

1.5.5 Risk prediction by individual and combined risk scores for CRC

In the prospective set, predictive performance of the miR-score was measured using AUC and Brier score. Potential over-optimism was accounted for by applying the 0.632+ bootstrapping method with 1000 replications. In addition to exploring the predictive ability of the miR-score over the entire period of follow-up, analyses were repeated with follow-up time restricted to the initial three years after recruitment and to subsequent years. Among participants with all scores available, predictive performance was evaluated for individual miR-score, ERS and PRS as well as different combinations of the scores using AUCs and Brier scores. For the individual miR-score, as well as the score combinations including miR-score, .632+ bootstrap was applied to adjust for potential over-estimation of predictive performance.

1.5.6 Deregulation of individual informative miRNAs

For the prospective set, we performed Mann-Whitney test to compare expression levels of individual informative miRNAs between cases and controls. We adjusted the P values to the number of comparisons using the Bonferroni-Holm method. The relative expression levels were calculated using $2^{-\Delta Ct}$ method. The correlation of expression levels of individual informative miRNAs across participants of the prospective set was assessed by Spearman correlation coefficients.

All statistical analyses were performed with statistical software R (version 3.6.1) (R Core Team, 2016), together with R packages 'mice' (version 3.12.0), 'ModelGood' (version 1.0.9) and 'pROC' (version 1.16.2). For all tests, two-sided P values of 0.05 or less were considered to be statistically significant.

Example 2: Results

2.1 Characteristics of study populations

The characteristics of populations from the discovery and prospective sets are shown in Table 1. The discovery set included 20 newly diagnosed CRC cases (from the GEKKO (Gebt dem Krebs keine Chance-Onkocheck) study arm B) and 20 controls free of colorectal neoplasm (from the GEKKO study arm A) matched by age and sex. Of the 19 cases with information about tumor stage at diagnosis, one was classified as stage 0, one as stage I, nine as stage II, four as stage III, and four as stage IV. The prospective set included 198 participants with incident CRC and 178 randomly selected participants without diagnosis of CRC identified within 14 years of follow-up in the ESTHER (Epidemiologische Studie zu Chancen der Verhütung, Früherkennung und optimierter Therapie chronischer Erkrankungen in der älteren Bevölkerung) study. By the 8-year follow-up, 62 cases (31.3%) and 95 controls (53.4%) had reported to have ever undergone a screening colonoscopy. For the incident cases, the time between sample collection and diagnosis ranged from 0.0 to 14.3 years (median (interquartile range), 6.8 (3.3-9.6) years). Of the 153 cases with information about tumor stage at diagnosis, 14 were classified as stage 0, 20 as stage I, 61 as stage II, 30 as stage III, and 28 as stage IV. Information on 140 relevant single-nucleotide polymorphisms (SNPs) used to build PRS was not available for 21 participants (cases n = 17; controls, n = 4). Therefore, the study population for the analyses on all scores (n= 355, cases n=181; controls, n=174) was smaller than the overall prospective set population. The distribution of characteristics was largely similar across both, the discovery and prospective sets with the mean age at sampling being around 65 years and males representing >50% of population in both sets.

2.2 Selection of miRNA candidates

In the discovery phase, we identified and selected 20 miRNAs differentially expressed from next-generation sequencing (NGS) profiling of discovery set samples (Supplementary Table 1) and 21 miRNAs reported to be differentially expressed in the literature (Supplementary Table 2) for quantitative real-time polymerase chain reaction (qPCR) profiling in the prospective set.

2.3 qPCR quality controls

RNA extraction efficiency, monitored using UniSp2 and UniSp4, was acceptable with raw quantification cycle (Cq) values being consistent across the dataset (UniSp2: Cq 21.26 $\pm$ 1.93, UniSp4: Cq 28.42 $\pm$ 2.82). UniSp6 was used to monitor the complementary DNA (cDNA) synthesis reactions and indicated constant efficiency of the reverse transcription step with no signs of inhibition (Cq 18.15 $\pm$ 0.11). Ten samples displayed significant hemolysis (mean $Cq_{miR-23a}$-mean $Cq_{miR-451a}$>7) and were excluded from downstream analysis (Figure 1). Development of the microRNA risk score (miR-score) in the prospective set.

Of the 41 miRNAs evaluated, three (miR-93-5p, miR-1246 and miR-223-3p) were selected as normalizers. Of the remaining 38 candidate miRNAs, seven were detectable (Cq value <40) in at least 99% of the samples and were identified as informative miRNAs. Samples with missing values for any of the informative miRNAs were excluded from further analysis (n= 9) and the remaining were included in the prospective set. The data was normalized to the average Cq value of the normalizers. The informative miRNAs were introduced as a panel into a logistic regression model on CRC risk, based on the prospective set. Using the observed weights from the regression model, a miR-score was calculated for each participant (linear predictor):

$$miR\text{-}score = 0.1899 + let\text{-}7g\text{-}5p*0.2351 + miR\text{-}19a\text{-}3p*\text{-}0.2024 + miR\text{-}23a\text{-}3p*1.6595 + miR\text{-}92a\text{-}3p*0.4794 + miR\text{-}144\text{-}5p*0.2002 + miR\text{-}21\text{-}5p*\text{-}1.6772 + miR\text{-}27a\text{-}3p*0.1014$$

2.4 Associations of the risk scores with CRC incidence

The associations of the risk scores with CRC incidence in the subpopulation including prospective set participants with all scores available are presented in Table 2. The middle quintiles (Q3) were assigned as the reference group in each set. For model 1, having a miR-score in the fifth quintile (Q5) was associated with a significantly increased risk of CRC [odds ratio (OR), 7.20 (95% confidence interval (CI), 3.60- 14.39)]. Additionally, having a miR-score in the first quintile (Q1) was associated with a significantly decreased risk of CRC [OR, 0.33 (95% CI, 0.12- 0.95)]. For model 2, with additional adjusting for age and sex, the miR-score remained a strong and significant predictor with an OR (95% CI) of 7.20 (3.56- 14.59). Associations for the ERS were much weaker and did not reach statistical significance. However, for model 1, having a PRS in the first quintile (Q1) was associated with a significantly decreased risk of CRC [OR, 0.46 (95% CI, 0.21- 0.97)]. Nevertheless, with additional adjusting for age and sex in model 2, the association did not remain significant.

2.5 Risk prediction by individual and combined risk scores for CRC

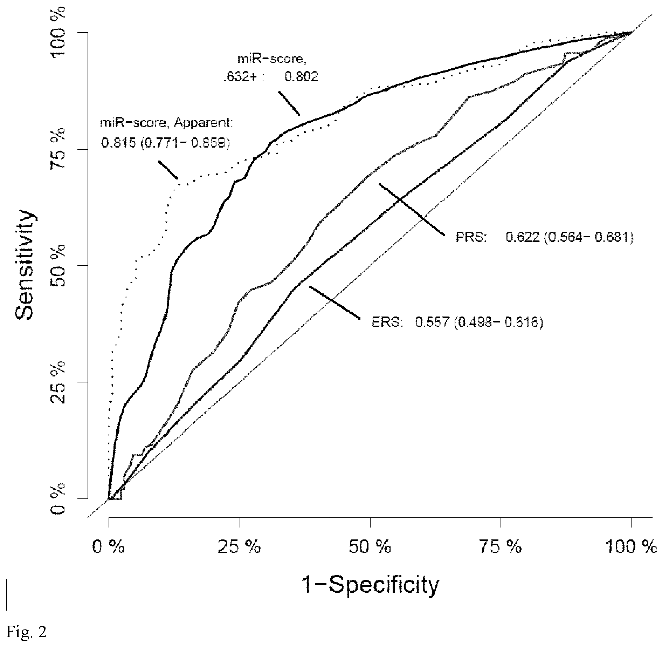

The predictive performances of individual risk scores and score combinations are presented in Table 3. In the prospective set, the miR-score showed a high prediction performance with an optimism-corrected area under the receiver-operating-characteristic curve (AUC) of 0.794 and a Brier score of 0.184. Additionally, consistent performance was observed in specific subgroups defined by follow-up time restricted to the initial three years after recruitment and to subsequent year. In the subpopulation including prospective set participants with all scores available, AUC was the lowest for ERS alone and the PRS performed slightly better than ERS (Figure 2). Combining PRS with ERS improved the predictive performance to a very limited extent [$AUC_{ERS + PRS}$ = 0.631 (95% CI, 0.573- 0.689) vs. $AUC_{ERS}$ = 0.557 (95% CI, 0.498 - 0.616) and $AUC_{PRS}$ = 0.622 (95% CI, 0.564- 0.681)]. The miR-score [optimism-corrected AUC = 0.802] substantially outperformed ERS, PRS and their combination. Compared to the model based on miR-score alone, models combining miR-score with ERS, or PRS, or both yielded optimism-corrected AUCs of 0.814, 0.822, and 0.820, respectively and resulted only in a minimal increase in performance.

2.6 Deregulations of individual informative miRNAs in the prospective set: comparison with discovery set and literature results

In the prospective set, evaluation of fold changes and corresponding P values of each individual informative miRNA (Table 4) revealed upregulation of three miRNAs (let-7g-5p, miR-23a-3p, and miR-92a-3p) in CRC cases versus controls.. Upregulation of miR-144-5p was consistent with our discovery set results. However, we observed downregulation of miR-19a-3p and miR-21-5p in CRC cases versus controls, which have been previously reported to be upregulated in other studies (Marcuello et al. (2019), Wikberg et al. (2018), Wang et al. (2014), Zheng et al. (2014), Giraldez et al (2013), Pan et al. (2017), Toiyama et al. (2013), Zanutto et al (2014), Zhu et al. (2017)). Furthermore, we observed downregulation of miR-27a-3p, previously reported to be upregulated in a study by Vychytilova-Faltejskova et al. (2016), but downregulated in a study by Tan et al. (2019). High significant positive correlation ($r_s$ = 0.73) was observed between miR-21-5p and miR-27a-3p.

2.7 Evaluation of panel with a lower number of miRNAs

Examplary panels with a lower number of miRNAs were evaluated; results are shown in Table 5.

2.8 Robustness of results

The score as specified above was tested for robustness by replacing single coefficients (weighting factors) by their respective vales $\pm$the standard error (SE) of the respective value. The SE values are shown in Table 6, results of the tests in Table 7.

Table 1: Characteristics of the study populations; Abbreviations: CRC, colorectal cancer; n, number; SD, standard deviation; TNM, Tumour Nodes Metastasis classification.

| Characteristics | Discovery set | | Characteristics | Prospective set | |
| --- | --- | --- | --- | --- | --- |
| | CRC Cases (n = 20) | Controls (n = 20) | | CRCCases (n = 198) | Controls (n = 178) |
| Age at sampling<br><br>Mean (SD)<br><br>Median (range) | 64.8 (12.3)<br><br>64 (47-88) | 64.7(12.1)<br><br>64 (47-87) | Age at sampling<br>Mean (SD)<br>Median (range) | 64.6(5.9)<br>65 (50-75) | 62.2(6.6)<br>62 (50-75) |
| | | | Age at diagnosis<br>Mean (SD)<br>Median (range) | 71.3 (6.8)<br>71.3 (53-86) | -<br>- |
| Gender- counts (%)<br>Male<br><br>Female | 11 (55.0)<br><br>9 (45.0) | 11 (55.0)<br><br>9 (45.0) | Gender- counts (%)<br>Male<br><br>Female | 122 (61.6)<br><br>76 (38.4) | 89 (50.0)<br><br>89 (50.0) |
| TNM stage- counts (%)<br>Stage 0<br>Stage I<br>Stage II<br>Stage III<br>Stage IV<br>Unknown | 1(5.0)<br>1(5.0)<br>9(45.0)<br>4(20.0)<br>4(20.0)<br>1 (5.0) | -<br>-<br>-<br>-<br>-<br>- | TNM stage at diagnosis-counts (%)<br>Stage 0<br>Stage I<br>Stage II<br>Stage III<br>Stage IV<br>Unknown | 14(7.1)<br>20(10.1)<br>61(30.8)<br>30(15.2)<br>28(14.1)<br>45 (22.7) | -<br>-<br>-<br>-<br>-<br>- |

Table 2. Individual associations of miR-score, ERS and PRS with CRC incidence in prospective set participants with all scores available ($N_{cases/controls}$ = 181/174); [a] Quintiles of risk score among controls; [b] Model 1: without adjustment for any confounders; [c] Model 2: like model 1, adjusted for age and sex; [d] OR, 95% CI and two-sided *P* values were generated from logistic regression model; Abbreviations: miR-score, microRNA risk score; ERS, environmental risk score; PRS, polygenic risk score; CRC, colorectal cancer; OR, odds ratio; CI, confidence interval; Q, quintile; Ref., reference category.

| Population | Quintile[a] | Cases | Controls | Model 1[b] | | Model 2[c] | |
|---|---|---|---|---|---|---|---|
| | | | | OR (95% CI)[d] | *P* value[d] | OR (95% CI)[d] | *P* value[d] |
| miR-score | Q1 (< -1.38] | 6 (3.3) | 36 (20.7) | 0.33 (0.12-0.95) | 0.0388 | 0.34 (0.12-0.97) | 0.0439 |
| | Q2 (-1.38, -0.91] | 14 (7.7) | 34 (19.5) | 0.82 (0.35-1.93) | 0.6552 | 0.88 (0.37-2.10) | 0.7759 |
| | Q3 (-0.91, -0.41] | 17 (9.4) | 34 (19.5) | Ref. | | Ref. | |
| | Q4 (-0.41, 0.06] | 18 (9.9) | 35 (20.1) | 1.03 (0.46-2.32) | 0.9459 | 1.09 (0.48-2.50) | 0.8352 |
| | Q5 (> 0.06) | 126 (69.6) | 35 (20.1) | 7.20 (3.60-14.39) | 2.28E-08 | 7.20 (3.56-14.59) | 4.19E-08 |
| ERS | Q1 (<3] | 34 (18.8) | 42 (24.1) | 0.83 (0.44-1.59) | 0.580 | -- | -- |
| | Q2 (3, 4] | 30 (16.6) | 34 (19.5) | 0.91 (0.46-1.78) | 0.779 | -- | -- |
| | Q3 (4, 5] | 35 (19.3) | 36 (20.7) | Ref. | | -- | -- |
| | Q4 (5, 7] | 46 (25.4) | 34 (19.5) | 1.39 (0.73-2.65) | 0.314 | -- | -- |
| | Q5 (>7) | 36 (19.9) | 28 (16.1) | 1.32 (0.67-2.61) | 0.419 | -- | -- |
| PRS | Q1 (<7.66] | 16 (8.8) | 35 (20.1) | 0.46 (0.21-0.97) | 0.042 | 0.49 (0.22-1.05) | 0.0680 |
| | Q2 (7.66, 7.92] | 24 (13.3) | 34 (19.5) | 0.71 (0.35-1.42) | 0.331 | 0.76 (0.37-1.56) | 0.4543 |
| | Q3 (7.92, 8.15] | 35 (19.3) | 35 (20.1) | Ref. | | Ref. | |
| | Q4 8.15, 8.46 | 47 (26.0) | 35 (20.1) | 1.34 (0.71 - 2.55 | 0.367 | 1.24 (0.64-2.40 | 0.5250 |
| | Q5 (>8.46) | 59 (32.6) | 35 (20.1) | 1.69 (0.90-3.16) | 0.103 | 1.75 (0.92-3.34) | 0.0880 |

Table 3. Risk prediction by individual and combined risk scores for CRC; Abbreviations: miR-score, microRNA risk score; ERS, environmental risk score; PRS, polygenic risk score; CRC, colorectal cancer; AUC, area under the receiver-operating-characteristic curve; CI, confidence interval. Note- miR-score was derived in the overall prospective set ($N_{cases/controls}$= 198/178).

| Population | Predictor | AUC (95% CI) | Brier score |
|---|---|---|---|
| Prospective set ($N_{cases/controls}$ = 198/178) | miR-score | Apparent: 0.808 (0.765-0.851); 0.632+: 0.794 | Apparent: 0.175; 0.632+: 0.184 |
| Prospective set participants with all scores available ($N_{cases/controls}$= 181/174) | ERS | 0.557 (0.498- 0.616) | 0.248 |
| | PRS | 0.622 (0.564- 0.681) | 0.240 |
| | ERS + PRS | 0.631 (0.573- 0.689) | 0.238 |
| | miR-score | Apparent: 0.815 (0.771-0.859); 0.632+: 0.802 | Apparent: 0.172; 0.632+: 0.181 |
| | ERS + miR-score | Apparent: 0.815 (0.771-0.859); 0.632+: 0.814 | Apparent: 0.172; 0.632+: 0.174 |
| | PRS + miR-score | Apparent: 0.824 (0.782-0.867); 0.632+: 0.822 | Apparent: 0.169; 0.632+: 0.171 |
| | ERS + PRS + miR-score | Apparent: 0.824 (0.781-0.867); 0.632+: 0.820 | Apparent: 0.169; 0.632+: 0.172 |

Table 4. Deregulation of each individual miRNA from the miR-score; [#] Values were generated from two-sided Mann-Whitney test [$] Multiple testing correction by the method of Bonferroni-Holm; ↑ represents significant up-regulation; ↓ represents significant down-regulation

| | Results in the Prospective set ($N_{cases/controls}$ = 198/178) | | | |
|---|---|---|---|---|
| | Deregulation | Fold change | *P* value[#] | Corrected *P* value[$] |
| let-7g-5p | ↑ | 1.26 | 5.04E-06 | 2.02E-05 |
| miR-19a-3p | ↓ | 0.61 | 2.01E-14 | 1.41E-13 |
| miR-23a-3p | ↑ | 1.21 | 1.26E-05 | 3.77E-05 |
| miR-92a-3p | ↑ | 1.11 | 2.72E-02 | 2.72E-02 |
| miR-144-5p | ↑ | 1.53 | 7.88E-08 | 3.94E-07 |
| miR-21-5p | ↓ | 0.69 | 2.94E-10 | 1.76E-09 |
| miR-27a-3p | ↓ | 0.82 | 3.74E-05 | 7.48E-05 |

Table 5: AUC estimates for panels with a reduced number of miRNAs

| | Predictor | AUC (0.632+) |
|---|---|---|
| 7-miRNA score | 0.1899 + let-7g-5p*0.2351 + miR-19a-3p*-0.2024 + miR-23a-3p*1.6595 + miR-92a-3p*0.4794 + miR-144-5p*0.2002 + miR-21-3p*-1.6772 + miR-27a-3p*0.1014 | 0.794 |
| | | |

(continued)

| | Predictor | AUC (0.632+) |
|---|---|---|
| 2-miRNA panels | 0.0008858 + miR.23a.3p*2.1009316 + miR.21.5p*-1.7322791 | 0.798 |
| | 1.4947 + miR.19a.3p*-1.1383 + miR.144.5p*0.5399 | 0.752 |
| | 1.0894 + let.7g.5p*0.7303 + miR.19a.3p*-1.1319 | 0.745 |
| | | |
| Individual miRNAs | miR-19a-3p | 0.728 |
| | miR-21-5p | 0.688 |
| | miR-144-5p | 0.660 |

Table 6: Standard Error (SE) estimates for coefficients

Coefficients:

| | Estimate | Std. Error | Coeff.+SE | Coeff. -SE |
|---|---|---|---|---|
| (Intercept) | 0.1899 | 0.9671 | 1.157 | -0.7772 |
| hsa.let.7g.5p | 0.2351 | 0.2478 | 0.4829 | -0.0127 |
| hsa.miR.19a.3p | -0.2024 | 0.2686 | 0.0662 | -0.471 |
| hsa.miR.23a.3p | 1.6595 | 0.4189 | 2.0784 | 1.2406 |
| hsa.miR.92a.3p | 0.4794 | 0.2627 | 0.7421 | 0.2167 |
| hsa.miR.144.5p | 0.2002 | 0.1507 | 0.3509 | 0.0495 |
| hsa.miR.21.5p | -1.6772 | 0.3462 | -1.331 | -2.0234 |
| hsa.miR.27a.3p | 0.1014 | 0.2694 | 0.3708 | -0.168 |

Table 7: AUC estimates for variant scores

| Score | Predictor | AUC (0.632+) |
|---|---|---|
| 7-miRNA score | 0.1899 + let-7g-5p*0.2351 + miR-19a-3p*-0.2024 + miR-23a-3p*1.6595 + miR-92a-3p*0.4794 + miR-144-5p*0.2002 + miR-21-5p*-1.6772 + miR-27a-3p*0.1014 | 0.794 |
| +1 S.E. for coefficient of let-7g-5p | 0.1899 + let-7g-5p*0.4829 + miR-19a-3p*-0.2024 + miR-23a-3p*1.6595 + miR-92a-3p*0.4794 + miR-144-5p*0.2002 + miR-21-5p*-1.6772 + miR-27a-3p*0.1014 | 0.805 |
| -1 S.E. for coefficient of let-7g-5p | 0.1899 + let-7g-5p*-0.0127 + miR-19a-3p*-0.2024 + miR-23a-3p*1.6595 + miR-92a-3p*0.4794 + miR-144-5p*0.2002 + miR-21-5p*-1.6772 + miR-27a-3p*0.1014 | 0.808 |
| +1 S.E. for coefficient of miR-19a-3p | 0.1899 + let-7g-5p*0.2351 + miR-19a-3p*0.0662 + miR-23a-3p*1.6595 + miR-92a-3p*0.4794 + miR-144-5p*0.2002 + miR-21-5p*-1.6772 + miR-27a-3p*0.1014 | 0.807 |
| -1 S.E. for coefficient of miR-19a-3 | 0.1899 + let-7g-5p*0.2351 + miR-19a-3p*-0.471 + miR-23a-3p*1.6595 + miR-92a-3p*0.4794 + miR-144-5p*0.2002 + miR-21-5p*-1.6772 + miR-27a-3p*0.1014 | 0.805 |
| +1 S.E. for coefficient of miR-23a-3p | 0.1899 + let-7g-5p*0.2351 + miR-19a-3p*-0.2024 + miR-23a-3p*2.0784 + miR-92a-3p*0.4794 + miR-144-5p*0.2002 + miR-21-5p*-1.6772 + miR-27a-3p*0.1014 | 0.805 |

(continued)

| Score | Predictor | AUC (0.632+) |
|---|---|---|
| -1 S.E. for coefficient of miR-23a-3p | 0.1899 + let-7g-5p*0.2351 + miR-19a-3p*-0.2024 + miR-23a-3p*1.2406 + miR-92a-3p*0.4794 + miR-144-5p*0.2002 + miR-21-5p*-1.6772 + miR-27a-3p*0.1014 | 0.803 |
| +1 S.E. for coefficient of miR-92a-3p | 0.1899 + let-7g-5p*0.2351 + miR-19a-3p*-0.2024 + miR-23a-3p*1.6595 + miR-92a-3p*0.7421 + miR-144-5p*0.2002 + miR-21-5p*-1.6772 + miR-27a-3p*0.1014 | 0.806 |
| -1 S.E. for coefficient of miR-92a-3p | 0.1899 + let-7g-5p*0.2351 + miR-19a-3p*-0.2024 + miR-23a-3p*1.6595 + miR-92a-3p*0.2167 + miR-144-5p*0.2002 + miR-21-5p*-1.6772 + miR-27a-3p*0.1014 | 0.807 |
| +1 S.E. for coefficient of miR-144-5p | 0.1899 + let-7g-5p*0.2351 + miR-19a-3p*-0.2024 + miR-23a-3p*1.6595 + miR-92a-3p*0.4794 + miR-144-5p*0.3509 + miR-21-5p*-1.6772 + miR-27a-3p*0.1014 | 0.806 |
| -1 S.E. for coefficient of miR-144-5p | 0.1899 + let-7g-5p*0.2351 + miR-19a-3p*-0.2024 + miR-23a-3p*1.6595 + miR-92a-3p*0.4794 + miR-144-5p*0.0495 + miR-21-5p*-1.6772 + miR-27a-3p*0.1014 | 0.808 |
| +1 S.E. for coefficient of miR-21-5p | 0.1899 + let-7g-5p*0.2351 + miR-19a-3p*-0.2024 + miR-23a-3p*1.6595 + miR-92a-3p*0.4794 + miR-144-5p*0.2002 + miR-21-5p* -1.331 + miR-27a-3p*0.1014 | 0.802 |
| -1 S.E. for coefficient of miR-21-5p | 0.1899 + let-7g-5p*0.2351 + miR-19a-3p*-0.2024 + miR-23a-3p*1.6595 + miR-92a-3p*0.4794 + miR-144-5p*0.2002 + miR-21-5p* -2.0234 + miR-27a-3p*0.1014 | 0.805 |
| +1 S.E. for coefficient of miR-27a-3 | 0.1899 + let-7g-5p*0.2351 + miR-19a-3p*-0.2024 + miR-23a-3p*1.6595 + miR-92a-3p*0.4794 + miR-144-5p*0.2002 + miR-21-5p*-1.6772 + miR-27a-3p*0.3708 | 0.808 |
| -1 S.E. for coefficient of miR-27a-3 | 0.1899 + let-7g-5p*0.2351 + miR-19a-3p*-0.2024 + miR-23a-3p*1.6595 + miR-92a-3p*0.4794 + miR-144-5p*0.2002 + miR-21-5p*-1.6772 + miR-27a-3p*-0.168 | 0.802 |
| +1 S.E. for coefficient of Intercept | 1.157 + let-7g-5p*0.2351 + miR-19a-3p*-0.2024 + miR-23a-3p*1.6595 + miR-92a-3p*0.4794 + miR-144-5p*0.2002 + miR-21-5p*-1.6772 + miR-27a-3p*0.1014 | 0.807 |
| -1 S.E. for coefficient of Intercept | -0.7772 + let-7g-5p*0.2351 + miR-19a-3p*-0.2024 + miR-23a-3p*1.6595 + miR-92a-3*0.4794 + miR-144-5p*0.2002 + miR-21-5p*-1.6772 + miR-27a-3p*0.1014 | 0.807 |
| Losing Intercept | let-7g-5p*0.2351 + miR-19a-3p*-0.2024 + miR-23a-3p*1.6595 + miR-92a-3p*0.4794 + miR-144-5p*0.2002 + miR-21-5p*-1.6772 + miR-27a-3p*0.1014 | 0.807 |

Literature:

Bader et al. (2020), IUBMB Life 72(2):275;
Bartel (2004), Cell 2004;116:281;
Carr et al. (2020), Gastroenterology 159(1): 129 ;
Carr et al. (2018), Gastroenterology 155(6): 1805);
Carter et al. (2017), Br J Cancer 116(6):762;
Chang et al. (2016), Oncotarget 7(9):10663;
Erben et al. (2021), Cancer Prev Res 14(6): 649;
Giraldez et al. (2013), Clin Gastroenterol Hepatol 11(6):681;

Huang et al (2010), Int J Cancer 127(1):118;
Jeon et al. (2018) Gastroenterology 154(8):2152;
Kurlapska et al. (2015), Clin Genet, 88(3):234;
Marcuello et al. (2019) Cancers 11(10): 1542
Ng et al. (2009), Gut 58(10):1375;
Pan et al. (2017), Oncotarget 8(40):68317;
Rodriguez-Montes et al. (2014), Cir Esp, 92(10):654;
Schetter et al. (2012), Cancer Journal 18(3):244;
Strubberg et al. (2017), Dis Model Mech, 10(3):197;
Sun et al. (2016), Oncotarget 7(10): 11434;
Tan et al. (2019), Cancer Epidemiol 60:67;
Thomas et al. (2020), The American Journal of Human Genetics 107(3):432;
Toiyama et al. (2013), J Natl Cancer Inst 105(12):849;
Toiyama et al (2018) Biochim Biophys Acta Rev Cancer 1870(2):274;
Vychytilova-Faltejskova et al. (2016), Carcinogenesis 37(10):941;
Wang et al. (2014) PLoS One 9(4):e87451;
Wikberg et al. (2018) Cancer Med 7(5):1697
Zanutto et al. (2014), Br J Cancer 110(4):1001;
Zanutto et al. (2020), Int J Cancer 146(4):1164
Zheng et al. (2014), Br J Cancer 111(10):198;
Zhu et al. (2017), Oncotarget 8(10):17081;

## Claims

1. A method for identifying a subject at risk of developing cancer, said method comprising

   (a) determining in a sample of said subject the amount of at least one miRNA selected from the list consisting of miR-19a-3p, let-7g-5p, miR-23a-3p, miR-92a-3p, miR-144-5p, and miR-27a-3p;
   (b) comparing said amount or amounts determined in step (a) to a reference or to references; and
   (c) identifying a subject at risk of developing cancer based on the result of comparing step (b).

2. The method of claim 1, wherein said cancer is colorectal cancer (CRC).

3. The method of claim 1 or 2, wherein said subject is CRC-free, preferably is cancer-free.

4. The method of any one of claims 1 to 3, wherein said at least one miRNA is miR-19a-3p or miR-144-5p.

5. The method of any one of claims 1 to 4, wherein at least two miRNAs are determined and wherein said at least two miRNAs are (i) miR-19a-3p and miR-144-5p or are (ii) miR-19a-3p and let-7g-5p.

6. The method of any one of claims 1 to 5, wherein said method further comprises determining the amount of miR-21-5p in step (a) and, preferably, comparing said amount to a reference in step (b).

7. The method of claim 6, wherein at least two miRNAs are determined and wherein said at least two miRNAs are miR-21-5p and miR-23a-3p.

8. The method of any one of claims 1 to 9, wherein the reference in step (b) is derived from a population of apparently healthy subjects or a population of subjects known not to have developed cancer; preferably wherein an amount of miRNA deviating from said reference is indicative of a subject at risk of developing cancer.

9. The method of any one of claims 6 to 8, wherein the amounts of miR-19a-3p, let-7g-5p, miR-23a-3p, miR-92a-3p, miR-144-5p, miR-21-5p and miR-27a-3p are determined and wherein said score is calculated according to equation (1)

$$\text{score}=w1*\text{miR-19a-3p} + w2*\text{let-7g-5p} + w3*\text{miR-23a-3p} + w4*\text{miR-92a-3p} + w5*\text{miR-144-5p} + w6*\text{miR-21-5p} + w7*\text{miR-27a-3p} \quad (1);$$

preferably wherein w1=-0.2 ± 0.3; w2 = 0.2 ± 0.3; w3 = 1.7 ± 0.5; w4 = 0.5 ± 0.3; w5 = 0.2 ± 0.2; w6 = -1.7 ± 0.4; and/or w7 = 0.1 ± 0.3.

10. The method of any one of claims 6 to 8, wherein said score is calculated according to equation (3)

$$\text{score} = 0.1899 + \text{let-7g-5p}*0.2351 + \text{miR-19a-3p}*-0.2024 + \text{miR-23a-3p}*1.6595 + \text{miR-92a-3p}*0.4794 + \text{miR-144-5p}*0.2002 + \text{miR-21-5p}*-1.6772 + \text{miR-27a-3p}*0.1014 \ (3).$$

11. The method of any one of claims 6 to 10, wherein said reference in step (b) is a reference score derived from a population of apparently heathy subjects or a population of subjects known not to develop cancer, wherein said reference is the lowest value of the upper quintile of scores in said population.

12. A method of identifying a subject not at risk of developing CRC comprising steps a) and b) of the method according to any one of claims 1 to 11, and step c1) identifying a subject not at risk of developing CRC based on the result of comparing step (b).

13. A kit comprising means for determining the amounts of at least six, more preferably all seven, miRNAs selected from the list consisting of miR-19a-3p, let-7g-5p, miR-23a-3p, miR-92a-3p, miR-144-5p, miR-21-5p and miR-27a-3p, preferably comprised in a housing.

14. A device comprising an evaluation unit with a processor, and, preferably tangibly embedded, instructions which, when performed on the processor, cause the device to perform at least step (b) of the method according to any one of claims 1 to 11.

15. A system comprising the a device according to claim 14 and an measuring unit adapted for measuring the amount of at least one miRNA specified in the method according to any one of claims 1 to 11.

Baseline participants of the ESTHER study
n= 9949

A sub-cohort consecutively enrolled
during the first six months of recruitment
n= 1000

Incident CRC cases
n= 219

Randomly selected CRC-free subjects until
the end of 2016
n= 200

Serum samples not available
n= 10

Hemolytic samples
n= 10

Samples in which any of the
normalizers was not
expressed; n= 14

Samples in which any of the
informative microRNAs was not
expressed; n=9

Incident CRC cases
n= 198

Randomly selected controls
n= 178

Fig. 1

Fig. 2

Europäisches
Patentamt

European
Patent Office

Office européen
des brevets

## EUROPEAN SEARCH REPORT

Application Number

EP 21 18 9450

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/184248 A1 (TSUCHIYA NAOTO [JP] ET AL) 2 July 2015 (2015-07-02) * paragraph [0098] – paragraph [0108] * ----- | 1-3,8, 11-13 | INV. C12Q1/6886 |
| X | US 2021/130904 A1 (SLABY ONDREJ [CZ] ET AL) 6 May 2021 (2021-05-06) * claim 1 * ----- | 1-3,8, 11,12 | |
| X | WO 2021/009184 A1 (INST DINVESTIGACIONS BIOMEDIQUES AUGUST PI I SUNYER IDIBAPS [ES] ET AL) 21 January 2021 (2021-01-21) * example 2.2 * ----- | 1-3,6,8, 11,12 | |
| X | WU CHUNG WAH ET AL: "Stool- and Blood-Based Molecular Tests in Screening for Colorectal Cancer: Ready for Prime Time?", CURRENT COLORECTAL CANCER REPORTS, SPRINGER US, BOSTON, vol. 13, no. 6, 10 November 2017 (2017-11-10), pages 481-488, XP036378747, ISSN: 1556-3790, DOI: 10.1007/S11888-017-0383-4 [retrieved on 2017-11-10] * the whole document * ----- | 1-3,8, 11,12 | |
| X | US 2019/316212 A1 (HERREROS VILLANUEVA MARTA [ES] ET AL) 17 October 2019 (2019-10-17) * example 2 * ----- -/-- | 1-4,8, 11,12 | TECHNICAL FIELDS SEARCHED (IPC) C12Q |

The present search report has been drawn up for all claims

| Place of search | Date of completion of the search | Examiner |
|---|---|---|
| The Hague | 14 January 2022 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 4**

Europäisches
Patentamt

European
Patent Office

Office européen
des brevets

# EUROPEAN SEARCH REPORT

Application Number

EP 21 18 9450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GAN XIA ET AL: "Blood-derived molecular signatures as biomarker panels for the early detection of colorectal cancer", MOLECULAR BIOLOGY REPORTS, SPRINGER NETHERLANDS, NL, vol. 47, no. 10, 26 September 2020 (2020-09-26), pages 8159-8168, XP037281092, ISSN: 0301-4851, DOI: 10.1007/S11033-020-05838-0 [retrieved on 2020-09-26] * page 8161, right-hand column * | 1-4,8, 11,12 | |
| X | ZHU MINGXIA ET AL: "A panel of microRNA signature in serum for colorectal cancer diagnosis", ONCOTARGET, vol. 8, no. 10, 7 March 2017 (2017-03-07), pages 17081-17091, XP055878742, DOI: 10.18632/oncotarget.15059 * page 17081 * | 1-4,8, 11,12 | |
| X | Zare Ali-Akbar ET AL: "This open-access article distributed under the terms of the Creative Commons Attribution NonCommercial 3.0 License (CC BY-NC 3.0). Downloaded from: www Tissue and plasma co-expression of microRNA let- 7g-5p in colorectal cancer patients", Journal of Medical Physiology, 1 January 2019 (2019-01-01), pages 1-6, XP055878845, Retrieved from the Internet: URL:http://jphysiology.com/index.php/jmp/article/view/49/42 [retrieved on 2022-01-13] * the whole document * | 1-3,8, 11,12 | TECHNICAL FIELDS SEARCHED (IPC) |

-/--

The present search report has been drawn up for all claims

| Place of search | Date of completion of the search | Examiner |
|---|---|---|
| The Hague | 14 January 2022 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 21 18 9450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OZCAN ONDER ET AL: "MTUS1 and its targeting miRNAs in colorectal carcinoma: significant associations", TUMOR BIOLOGY, KARGER, BASEL, CH, vol. 37, no. 5, 7 December 2015 (2015-12-07), pages 6637-6645, XP036218983, ISSN: 1010-4283, DOI: 10.1007/S13277-015-4550-4 [retrieved on 2015-12-07] * page 6643; table 3 * | 1-5,8, 11,12 | |
| X | WU CHUNG WAH ET AL: "Novel Approach to Fecal Occult Blood Testing by Assay of Erythrocyte-Specific microRNA Markers", DIGESTIVE DISEASES AND SCIENCES, SPRINGER NEW YORK LLC, US, vol. 62, no. 8, 28 June 2017 (2017-06-28), pages 1985-1994, XP036277929, ISSN: 0163-2116, DOI: 10.1007/S10620-017-4627-6 [retrieved on 2017-06-28] * page 1990 * | 1-4,8, 11,12 | |
| X,D | PETRA VYCHYTILOVA-FALTEJSKOVA ET AL: "Serum-based microRNA signatures in early diagnosis and prognosis prediction of colon cancer", CARCINOGENESIS, vol. 37, no. 10, 1 August 2016 (2016-08-01), pages 941-950, XP055435179, GB ISSN: 0143-3334, DOI: 10.1093/carcin/bgw078 * the whole document * | 1-3,6-8, 11,12 | TECHNICAL FIELDS SEARCHED (IPC) |

-/--

The present search report has been drawn up for all claims

| Place of search | Date of completion of the search | Examiner |
|---|---|---|
| The Hague | 14 January 2022 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## EUROPEAN SEARCH REPORT

Application Number

EP 21 18 9450

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/123614 A1 (CHOWDHURY DIPANJAN [US] ET AL) 23 April 2020 (2020-04-23) <br> * paragraph [0031]; claim 1; table 2 * <br> ----- | 1,8,11, 13-15 | |
| | | | **TECHNICAL FIELDS SEARCHED (IPC)** |

The present search report has been drawn up for all claims

| Place of search | Date of completion of the search | Examiner |
|---|---|---|
| The Hague | 14 January 2022 | Gabriels, Jan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...............................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 18 9450

14-01-2022

| Patent document cited in search report | | Publication date | Patent family member(s) | | Publication date |
|---|---|---|---|---|---|
| US 2015184248 | A1 | 02-07-2015 | JP | WO2014003053 A1 | 02-06-2016 |
| | | | US | 2015184248 A1 | 02-07-2015 |
| | | | WO | 2014003053 A1 | 03-01-2014 |
| US 2021130904 | A1 | 06-05-2021 | EP | 3431609 A1 | 23-01-2019 |
| | | | ES | 2785682 T3 | 07-10-2020 |
| | | | HU | E048369 T2 | 28-07-2020 |
| | | | JP | 2020527047 A | 03-09-2020 |
| | | | JP | 2021090451 A | 17-06-2021 |
| | | | PL | 3431609 T3 | 02-11-2020 |
| | | | US | 2021130904 A1 | 06-05-2021 |
| | | | WO | 2019015702 A1 | 24-01-2019 |
| WO 2021009184 | A1 | 21-01-2021 | NONE | | |
| US 2019316212 | A1 | 17-10-2019 | EP | 3327135 A1 | 30-05-2018 |
| | | | EP | 3545109 A1 | 02-10-2019 |
| | | | US | 2019316212 A1 | 17-10-2019 |
| | | | WO | 2018096154 A1 | 31-05-2018 |
| US 2020123614 | A1 | 23-04-2020 | EP | 3565903 A1 | 13-11-2019 |
| | | | US | 2020123614 A1 | 23-04-2020 |
| | | | WO | 2018129535 A1 | 12-07-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KURLAPSKA et al.** *Clin Genet,* 2015, vol. 88 (3), 234
- **JEON et al.** *Gastroenterology,* 2018, vol. 154 (8), 2152
- **BARTEL.** *Cell 2004,* 2004, vol. 116, 281
- **STRUBBERG et al.** *Dis Model Mech,* 2017, vol. 10 (3), 197
- **SCHETTER et al.** *Cancer Journal,* 2012, vol. 18 (3), 244
- **CARTER et al.** *Br J Cancer,* 2017, vol. 116 (6), 762
- **MARCUELLO et al.** *Cancers,* 2019, vol. 11 (10), 1542
- **RODRIGUEZ-MONTES et al.** *Cir Esp,* 2014, vol. 92 (10), 654
- **TOIYAMA et al.** *Biochim Biophys Acta Rev Cancer,* 2018, vol. 1870 (2), 274
- **SUN et al.** *Oncotarget,* 2016, vol. 7 (10), 11434
- **ZANUTTO et al.** *Int J Cancer,* 2020, vol. 146 (4), 1164
- **WIKBERG et al.** *Cancer Med,* 2018, vol. 7 (5), 1697
- **BADER et al.** *IUBMB Life,* 2020, vol. 72 (2), 275
- **CHANG et al.** *Oncotarget,* 2016, vol. 7 (9), 10663
- **GIRALDEZ et al.** *Clin Gastroenterol Hepatol,* 2013, vol. 11 (6), 681
- **HUANG et al.** *Int J Cancer,* 2010, vol. 127 (1), 118
- **NG et al.** *Gut,* 2009, vol. 58 (10), 1375
- **PAN et al.** *Oncotarget,* 2017, vol. 8 (40), 68317
- **TAN et al.** *Cancer Epidemiol,* 2019, vol. 60, 67
- **TOIYAMA et al.** *J Natl Cancer Inst,* 2013, vol. 105 (12), 849
- **VYCHYTILOVA-FALTEJSKOVA et al.** *Carcinogenesis,* 2016, vol. 37 (10), 941
- **WANG et al.** *PLoS One,* 2014, vol. 9 (4), e87451
- **ZANUTTO et al.** *Br J Cancer,* 2014, vol. 110 (4), 1001
- **ZHENG et al.** *Br J Cancer,* 2014, vol. 111 (10), 198
- **ZHU et al.** *Oncotarget,* 2017, vol. 8 (10), 17081
- **ERBEN et al.** *Cancer Prev Res,* 2021, vol. 14 (6), 649
- **CARR et al.** *Gastroenterology,* 2020, vol. 159 (1), 129
- **CARR et al.** *Gastroenterology,* 2018, vol. 155 (6), 1805
- **GRIFFITHS-JONES S.** *NAR,* 2004, vol. 32, D109-D111
- **KOZOMARA A ; GRIFFITHS-JONES S.** *NAR,* 2011, vol. 39, D152-D157
- **ZWEIG.** *Clin. Chem.,* 1993, vol. 39, 561
- **BARTEL.** *Cell,* 2004, vol. 116, 281
- **THOMAS et al.** *The American Journal of Human Genetics,* 2020, vol. 107 (3), 432